# EUROPÄISCHE PATENTSCHRIFT

(11) **EP 4 483 823 B1**
(45) Veröffentlichungstag und Bekanntmachung des Hinweises auf die Patenterteilung: **27.08.2025**
(21) Anmeldenummer: 23182397.2
(22) Anmeldetag: 29.06.2023
(51) Int. Cl.: A61B 17/34, A61B 90/13, A61B 34/10, A61B 90/00

(54) **MEDIZINISCHE EINGRIFFSVORRICHTUNG, COMPUTERIMPLEMENTIERTES VERFAHREN ZUR ERMITTLUNG UND AUSGABE EINES POSITIONIERHINWEISES UND COMPUTERPROGRAMM**
MEDICAL INTERVENTION DEVICE, COMPUTER-IMPLEMENTED METHOD FOR ASCERTAINING AND OUTPUTTING A POSITIONING INDICATION, AND COMPUTER PROGRAM
DISPOSITIF D'INTERVENTION MÉDICALE, PROCÉDÉ MIS EN OEUVRE PAR ORDINATEUR POUR DÉTERMINER ET DÉLIVRER UN INDICE DE POSITIONNEMENT ET PROGRAMME INFORMATIQUE

(43) Veröffentlichungstag der Anmeldung: 01.01.2025
(73) Patentinhaber: Siemens Healthineers AG, 91301 Forchheim (DE)
(72) Erfinder: Regensburger, Alois, 91099 Poxdorf (DE)
(74) Vertreter: Siemens Healthineers Patent Attorneys

(56) Entgegenhaltungen:
- DE-A1- 102019 211 870
- US-A1- 2008 208 041
- US-A1- 2015 150 591
- US-A1- 2020 315 711

## Beschreibung

Die Erfindung betrifft eine medizinische Eingriffsvorrichtung, aufweisend ein medizinisches, längliches Instrument, das für einen Eingriff in einen Eingriffsbereich eines Untersuchungsobjekts teilweise einführbar ist, einen proximalen, außerhalb des Untersuchungsobjekts anzuordnenden Anteil zur manuellen Handhabung des medizinischen Instruments aufweist und wenigstens teilweise biegbar ist. Daneben betrifft die Erfindung ein computerimplementiertes Verfahren zur Ermittlung und Ausgabe eines Positionierhinweises und ein Computerprogramm.

Längliche medizinische Instrumente, die zur Untersuchung und/oder Behandlung eines Patienten in den Patienten eingebracht werden, sind im Stand der Technik bekannt. Insbesondere sind hierbei Nadeln als medizinische Instrumente zu nennen, beispielsweise Biopsienadeln, Ablationsnadeln und dergleichen. Hierbei ist es üblicherweise vorgesehen, die Nadel innerhalb eines Untersuchungsobjekts derart zu bewegen, dass ihre Spitze einen Zielbereich, insbesondere ein Zielobjekt, innerhalb des Untersuchungsobjekts erreicht. Bei dem Zielbereich kann es sich beispielsweise um einen Tumor oder anderweitig interessierendes und/oder zu behandelndes Gewebe handeln. Um den Zielbereich verlässlich zu erreichen, insbesondere einen das Instrument manuell führenden Benutzer zu unterstützen, sind eine Vielzahl von Effekten zu berücksichtigen.

So ist beispielsweise zu beachten, dass dann, wenn ein Instrument, insbesondere eine Nadel, in ein Weichgewebe eingestochen wird, dieses Weichgewebe typischerweise nicht statisch bleibt. Einerseits kommt es zu Bewegungen im Untersuchungsobjekt, beispielsweise makroskopischen Bewegungen eines Patienten und/oder zyklischen Bewegungen, wie Atembewegungen und Herzschlag. Zum anderen kann auch das Instrument selbst zu Gewebeverschiebungen führen. So können beispielsweise Organe und/oder andere anatomische Strukturen zur Seite und/oder nach hinten gedrückt werden und/oder anderweitige Deformationen auftreten. Mithin verzichten Benutzer, beispielsweise interventionelle Radiologen, häufig auf eine Navigation bei der Nadelführung und führen die Nadel (bzw. das sonstige Instrument) schrittweise vorwärts, unterbrochen durch wiederholte Bildgebung, insbesondere Röntgenbildgebung, und Pfadkorrektur. Hierbei ist es beispielsweise bekannt, den proximalen Anteil, also den zum Benutzer hin zeigenden Anteil, des Instruments, das bereits teilweise eingestochen ist, zur Korrektur des Instrumentenpfads seitlich zu bewegen, um danach das Instrument wieder ein Stück vorzuschieben. Weicht der Pfad des Instruments zu sehr vom Gewollten ab, kann das Instrument ein Stück zurückgezogen und erneut korrigiert werden. Diese Korrekturen des Instrumentenpfads beruhen auf der Intuition und Erfahrung des Benutzers, wobei jede Korrektur des Instrumentenpfads das Gewebe des Untersuchungsobjekts beeinflusst, die Dauer des Eingriffs verlängert und aufgrund der wiederholten Bildaufnahmen durch Röntgenbildgebung die Strahlenbelastung erhöht.

Je nach Durchmesser, Material und Flexibilität des Instruments, also der mechanischen Eigenschaften, führt die seitliche Bewegung des proximalen Anteils des Instruments entweder zu einer starren Richtungsänderung des bereits teilweise eingestochenen Instruments innerhalb der Anatomie oder zu einer elastischen Verbiegung des Instruments, sodass gekrümmte Nadelpfade ermöglicht werden. Es existiert auch ein weiter Übergangsbereich, in dem sowohl Richtungsänderung als auch Verbiegung auftreten. All diese Abschätzungen bleiben bislang der Erfahrung und Intuition des Benutzers überlassen.

Die häufigste Vorgehensweise, die heute für Eingriffe mit medizinischen Instrumenten, insbesondere Nadeln, genutzt wird, ist das sogenannte "Step-and-Shoot-Verfahren", wie oben bereits dargelegt. Hierbei wird die Nadel schrittweise vorgeschoben und regelmäßig durch Röntgenbildgebung eine Positionskontrolle durchgeführt, wobei immer dann, wenn die Nadel in der aktuellen Richtung nicht zum Zielbereich führt, die Nadel entsprechend neu ausgerichtet wird. Anschließend wird wieder ein Stück vorgeschoben und ein neuer Kontrollscan durchgeführt. Dies benötigt allerdings eine große Erfahrung des Benutzers.

Vorgeschlagen wurde im Stand der Technik auch, eine Vorabplanung durchzuführen, worin eine statische und geradlinige Nadeltrajektorie, beispielsweise in einem dreidimensionalen Vorab-Bilddatensatz des Untersuchungsobjekts, geplant wird. Diese geplante Nadeltrajektorie kann zur Durchführung des Eingriffs per optischer Navigation, wobei beispielsweise gekreuzte Laserlinien die Nadeltrajektorie anzeigen können, visualisiert werden. Bei dieser Vorgehensweise erfolgt jedoch weder eine Anpassung an die gegebenenfalls veränderte Anatomie, noch wird eine Biegbarkeit des Instruments, insbesondere der Nadel, berücksichtigt.

Schließlich wurde im Stand der Technik bereits vorgeschlagen, das Einführen des Instruments, insbesondere der Nadel, durch einen Roboter durchzuführen. Hierbei wurde beispielsweise vorgeschlagen, dass ein Roboter eine Nadel schrittweise vorschiebt und ein Kontrollscan durch Computertomographie ausgeführt wird. Darin werden die aktuelle Position und Form sowie gegebenenfalls die aktuelle Lage des Zielbereichs innerhalb der Anatomie in den Bilddaten bestimmt, woraus ein angepasster Nadelpfad samt korrekter Verbiegung des außerhalb des Patienten befindlichen Anteils automatisch berechnet werden. Der Roboter wird dann gemäß dem angepassten Nadelpfad angesteuert. Nach mehrfacher Ausführung dieser Schritte gelangt die Nadel mittels an die Veränderung der Anatomie angepasster Verbiegung automatisch in den Zielbereich. Beispielsweise offenbart WO 2022/254436 A1 Systeme, Vorrichtungen und Verfahren zum Echtzeit-Update einer Trajektorie zum Einführen eines medizinischen Instruments zu einem Ziel in einem Körper eines Subjekts, wobei eine Steuerung gemäß eines geschlossenen Regelkreises hin zu dem bewegten Ziel erfolgt. Dokument US 2020/315711 A1 offenbart Systeme, Vorrichtungen und Verfahren zur Führung von Trajektorien unter Verwendung von Positionsdaten des Instruments und geplanten Trajektorien in oder durch den Körper eines Patienten oder ein Objekt. Laser werden von einem Lasersystem in Richtung des Körpers des Patienten oder des Objekts in einem Bereich innerhalb oder in der Nähe der Sichtlinie des Bedieners des Instruments emittiert. Informationen über das Instrument, einschließlich der Position seines proximalen und distalen Endes und der Position des Körpers oder Objekts des Patienten, werden verwendet, um die Emission der Laser in Echtzeit während des Betriebs des Instruments zu steuern. Die emittierten Laser dienen als visuelle Hinweise darauf, wie das distale und das proximale Ende des Instruments zu positionieren sind, um die geplante Trajektorie korrekt zu erreichen. Eine Führungskarte auf dem Instrument kann verwendet werden, um visuelle Hinweise auf die Position des distalen und proximalen Endes des Instruments relativ zu einer geplanten Trajektorie auszugeben.

Für die Verwendung von starren Schrauben wurde auch bereits vorgeschlagen, zur Unterstützung eines Benutzers eine Darstellung zu erzeugen, die durch Überlagerung von Bilddaten anzeigt, wohin die Schraube bewegt würde, wenn sie weiter geradeaus in das Untersuchungsobjekt vorgeschoben wird.

Der Erfindung liegt die Aufgabe zugrunde, einem Benutzer eine intuitive Hilfestellung beim manuellen Vorschub von medizinischen Instrumenten in einem Untersuchungsobjekt bereitzustellen, welche insbesondere ein schnelles und verlässliches Erreichen des Zielbereichs ermöglicht.

Diese Aufgabe wird erfindungsgemäß gelöst durch eine medizinische Eingriffsvorrichtung, ein computerimplementiertes Verfahren und ein Computerprogramm gemäß den unabhängigen Patentansprüchen. Vorteilhafte Weiterbildungen ergeben sich aus den abhängigen Patentansprüchen.

Die vorliegende Erfindung wird im Folgenden anhand der medizinischen Eingriffsvorrichtung, des Verfahrens sowie des Computerprogramms genauer erläutert. Dabei lassen sich die Ausführungen zu den jeweiligen Erfindungsgegenständen untereinander jeweils analog übertragen, sodass insbesondere Ausgestaltungen und Ausführungen zu der medizinischen Eingriffsvorrichtung auch auf das computerimplementierte Verfahren übertragen werden können sowie umgekehrt.

Eine erfindungsgemäße medizinische Eingriffsvorrichtung weist auf:
- ein medizinisches, längliches Instrument, das für einen Eingriff in einen Eingriffsbereich eines Untersuchungsobjekts teilweise einführbar ist, einen proximalen, außerhalb des Untersuchungsobjekts anzuordnenden Anteil zur manuellen Handhabung des medizinischen Instruments aufweist und wenigstens teilweise, insbesondere elastisch, biegbar ist,
- ein an dem Instrument angeordnetes und/oder in dieses integriertes Ausrichtungselement, das eine Projektionsfläche mit wenigstens einer Markierung, die wenigstens einen Punkt markiert, aufweist,
- eine Darstellungseinrichtung,
- eine Verarbeitungseinrichtung, welche aufweist:
   - eine Auswertungseinheit, welche dazu ausgebildet ist, aus den Eingriffsbereich darstellenden Bilddaten des Untersuchungsobjekts und die aktuelle Position und Lage des medizinischen Instruments beschreibenden Positionierungsdaten unter Verwendung von die Biegbarkeit des medizinischen Instruments beschreibenden Eigenschaftsdaten wenigstens eine prädiktive, insbesondere nichtlineare, Fortsetzungstrajektorie zu ermitteln, und
   - eine Ausgabeeinheit zur Ausgabe einer auf den Bilddaten basierenden Darstellung des Eingriffsbereichs und der wenigstens einen Fortsetzungstrajektorie auf der Darstellungseinrichtung; und
- eine Lichtführungseinrichtung zur Projektion wenigstens eines Lichtmusters auf die Projektionsfläche derart, dass durch Zusammenschau wenigstens einer der wenigstens einen Markierung und des Lichtmusters ein Positionierhinweis zur manuellen Positionskorrektur an dem proximalen Anteil des medizinischen Instruments zum Erreichen einer auszuwählenden der wenigstens einen Fortsetzungstrajektorie bei weiterem Einschub des medizinischen Instruments entsteht.

Das Untersuchungsobjekt kann beispielsweise eine menschliche und/oder tierische Patientin und/oder ein menschlicher und/ oder tierischer Patient und/oder ein Untersuchungsphantom sein. Eine erfindungsgemäße medizinische Eingriffsvorrichtung, die auch als medizinisches Eingriffssystem verstanden werden kann, weist mithin mehrere Komponenten auf, durch deren Zusammenwirken die Ausgabe von intuitiven, unterstützenden Positionierhinweisen erlaubt wird.

Das medizinische Instrument ist insbesondere ein chirurgisches Instrument und bevorzugt eine Nadel, insbesondere eine Punktionsnadel. Andere denkbare medizinische Instrumente umfassen Bohrer, Schrauben, diagnostische Instrumente, wie beispielsweise Endoskope und/oder Katheter, und/oder Trokare. Das medizinische Instrument ist länglich, mithin langgestreckt, und bevorzugt nadel- und/oder stabförmig. Im Betriebszustand der Vorrichtung ist das medizinische Instrument bevorzugt wenigstens teilweise innerhalb des Untersuchungsobjekts, konkret im Eingriffsbereich, angeordnet. Der innerhalb des Untersuchungsobjekts angeordnete Anteil des medizinischen Instruments wird vorliegend auch als distaler Anteil bezeichnet.

Das Ausrichtungselement kann in definierter Lagebeziehung, insbesondere in einer definierten räumlichen Relativposition und/oder Relativausrichtung und/oder Relativpose, an dem medizinischen Instrument, insbesondere dem proximalen Abschnitt des medizinischen Instruments, angeordnet sein. Das Ausrichtungselement und/oder das medizinische Instrument kann ein Befestigungselement aufweisen, beispielsweise eine Klemmvorrichtung und/oder eine Steckvorrichtung und/oder eine Magnethalterung. Alternativ oder zusätzlich kann das Ausrichtungselement wenigstens teilweise in das medizinische Instrument, insbesondere an dem proximalen Anteil, integriert sein, beispielsweise an einer Oberfläche des medizinischen Instruments.

Das Ausrichtungselement weist eine Projektionsfläche auf, auf welcher wenigstens eine Markierung vorgesehen ist. Abgesehen von der Markierung kann die Projektionsfläche insbesondere in einer bestimmten Farbe, beispielsweise weiß, eingefärbt sein. Die Projektionsfläche wird insbesondere durch wenigstens einen Teil der sichtbaren Oberfläche des Ausrichtungselements gebildet. Die wenigstens eine Markierung markiert zumindest einen Punkt und kann zumindest punktförmig sein. Die wenigstens eine Markierung kann als geometrisches Objekt, beispielsweise als eine Linie und/oder ein Kreis und/oder ein Kreuz, ausgebildet sein. Ferner kann die wenigstens eine Markierung als Kontrastobjekt, beispielsweise Aufdruck, und/oder Erhebung und/oder Aussparung an einem Substrat des Ausrichtungselements, insbesondere einer Oberfläche des Ausrichtungselements, ausgebildet sein.

Die Verarbeitungseinrichtung kann eine Schnittstelle zum Empfangen der Bilddaten (und gegebenenfalls der Positionierungsdaten) aufweisen. Bevorzugt ist die Auswertungseinheit ausgebildet, die Positionierungsdaten durch Auswertung der, insbesondere auch das medizinische Instrument in dem Eingriffsbereich zeigenden, Bilddaten zu ermitteln. Die Positionierungsdaten können jedoch auch zumindest teilweise anderweitig bereitgestellt werden, beispielsweise von einem die Position und/oder Orientierung des medizinischen Instruments anderweitig ermittelnden, mit den Bilddaten registrierten Positionsbestimmungssystem.

Über die Schnittstelle kann die Verarbeitungseinrichtung zum Empfangen der medizinischen Bilddaten ausgebildet sein. Das Empfangen der Bilddaten kann insbesondere ein Erfassen und/ oder Auslesen eines computerlesbaren Datenspeichers und/oder ein Empfangen aus einer Datenspeichereinheit, beispielsweise einer Datenbank, umfassen. Bevorzugt jedoch werden die Bilddaten von einer medizinischen Bildgebungseinrichtung, die auch Teil der Eingriffsvorrichtung sein kann, bereitgestellt.

Vorteilhafterweise umfassen die Bilddaten eine zweidimensional oder dreidimensional räumlich aufgelöste Abbildung des Untersuchungsobjekts. Zudem können die Bilddaten das Untersuchungsobjekt zeitaufgelöst abbilden. Die Bilddaten können mehrere Bildpunkte, insbesondere Pixel oder Voxel, mit Bildwerten, beispielsweise Intensitätswerten und/oder Schwächungswerten, aufweisen, welche das Untersuchungsobjekt abbilden, insbesondere repräsentieren. Bevorzugt umfassen die Bilddaten Röntgenbilddaten des Untersuchungsobjekts, insbesondere einen Computertomographie-Bilddatensatz und/oder Projektionsbilder, beispielsweise Fluoroskopiebilder. Zweckmäßigerweise können die Bilddaten auch das medizinische Instrument zumindest teilweise abbilden.

Die Darstellungseinrichtung kann einen Bildschirm und/oder einen Monitor und/oder einen Projektor und/oder eine Datenbrille umfassen, welche zur Anzeige der graphischen Darstellung ausgebildet sind.

Die Lichtführungseinrichtung kann vorteilhafter Weise eine Lichtquelle, insbesondere eine Laserlichtquelle, umfassen, welche dazu ausgebildet ist, das Lichtmuster auszusenden. Hierfür kann die Lichtführungseinrichtung beispielsweise eine optische Blende umfassen. Wie im Folgenden noch genauer ausgeführt werden wird, kann die Verarbeitungseinrichtung eine Ansteuereinheit zur Ansteuerung der Lichtführungseinrichtung aufweisen. Die Lichtführungseinrichtung kann insbesondere ausgebildet sein, wenigstens einen Laserfächer und/oder wenigstens einen Laserstrahl zur Projektion einer Linie bzw. eines Punktes auszusenden. Die Lichtführungseinrichtung kann wenigstens einen Aktor aufweisen, um eine Veränderung des Lichtmusters herbeizuführen. Die Ansteuerung durch die Ansteuereinheit kann den Aktor betreffen.

Ist das Untersuchungsobjekt auf einer Patientenliege gelagert, die verstellbar ist, kann die relative Position und Orientierung des Lichtmusters auch durch eine Ansteuerung einer Aktorik der Patientenliege erfolgen. Beispielsweise sind Ausgestaltungen bekannt, in denen zwei Laserfächer, mithin Linien im Lichtmuster, die Lage einer aufgenommenen (meist axialen) Schicht der Computertomographie und eine Mitte der Bildgebungseinrichtung, hier Computertomographieeinrichtung, anzeigen. Durch seitliche Verstellung der Patientenliege kann dann auch die Mittellinie wie gewünscht positioniert werden.

Die Verarbeitungseinrichtung kann insgesamt als Steuereinrichtung der Eingriffsvorrichtung dienen. Sie kann wenigstens ein Speichermittel und wenigstens einen Prozessor aufweisen. Funktionseinheiten, wie beispielsweise die Auswertungseinheit und die Ausgabeeinheit, können durch Hardware und/oder Software gebildet sein. Neben den genannten Funktionseinheiten sind selbstverständlich auch weitere Funktionseinheiten grundsätzlich denkbar.

Die Auswertungseinheit ist dazu ausgebildet, Fortsetzungstrajektorien zu ermitteln, die sich bei bestimmten Positionskorrekturen am proximalen Anteil und weiterem Vorschub ergeben. Während die Darstellung eine Bewertung der wenigstens einen Fortsetzungstrajektorie durch den Benutzer erlaubt, dient der Positionierhinweis dazu, den Benutzer anzuleiten, diese Positionskorrekturen für die (ausgewählte) Trajektorie vorzunehmen. Aufgrund der Berücksichtigung der (aktuellen) Bilddaten geht hierin der aktuelle Zustand der Anatomie des Untersuchungsobjekts im Eingriffsbereich ein. Erfindungsgemäß wird jedoch zudem auch nicht von einem starren medizinischen Instrument ausgegangen, sondern dem tatsächlich, insbesondere elastisch, biegsamen Instrument werden diese mechanischen Eigenschaften, die die Biegsamkeit erlauben, auch bei der Ermittlung der wenigstens einen Fortsetzungstrajektorie zugeordnet, sodass sich eine zuverlässige und genaue Vorhersage ergibt und sich gleichzeitig die Unterstützung des Benutzers bei der manuellen Führung des medizinischen Instruments, insbesondere der Nadel, auch auf eine bislang nur intuitiv durch den Benutzer berücksichtigte Eigenschaft bzw. einen Freiheitsgrad bezieht, nämlich die Biegbarkeit des Instruments. Hierdurch kann insbesondere bei der Ermittlung eine dreidimensionale Beurteilung erfolgen. Die Biegbarkeit des Instruments beschränkt sich dabei insbesondere nicht auf den proximalen Anteil, sondern auch auf den distalen, in den Patienten eingeführten bzw. einführbaren Anteil, so dass auch gebogene Formen im Patienten auftreten können/sollen.

Dabei kann konkret vorgesehen sein, dass die Auswertungseinheit zur Ermittlung der wenigstens einen Fortsetzungstrajektorie durch Simulation und/oder Modellierung der Auswirkung einer im proximalen Anteil vorgenommenen, insbesondere eine Verbiegung umfassenden, Positionskorrektur des medizinischen Instruments auf das Verhalten des Instruments am distalen Anteil innerhalb des Untersuchungsobjekts, insbesondere bei Vorschub, ausgebildet ist. Die Verbiegung im proximalen Anteil kann, wenn, wie im Folgenden zur ersten Ausführungsform noch näher erläutert werden wird, Markierungen bestimmten Fortsetzungstrajektorien zugeordnet werden sollen, so vorgegeben werden, dass die durch die jeweilige Markierung beschriebene Positionskorrektur verwendet wird. Alternativ oder zusätzlich ist es auch denkbar, dass der Auswertungseinheit eine Zielinformation über ein Instrumentenziel, insbesondere einen Zielbereich, in dem Eingriffsbereich bereitgestellt wird und diese ausgebildet ist, wenigstens eine Fortsetzungstrajektorie zur größtmöglichen Annäherung an dieses Instrumentenziel und eine hierfür nötige Positionskorrektur (insbesondere umfassend eine Verbiegung des proximalen Anteils) zu ermitteln. In der zweiten, im Folgenden noch konkret beschriebenen Ausführungsform kann ein Projektionspunkt dies dann anzeigen. In der ersten angesprochenen Ausführungsform kann beispielsweise eine nächstgelegene Markierung, die diese Positionskorrektur beschreibt, durch die Auswertungseinheit ermittelt werden. Eine Verbiegung am proximalen Anteil kann auch eine Verbiegung am distalen Anteil zur Folge haben.

In der Darstellung auf der Darstellungseinrichtung können die Fortsetzungstrajektorien, die als virtuelle Fortsetzungen verstanden werden können, beispielsweise als Bilddaten ortsgenau überlagert dargestellt werden. Hierbei können die Fortsetzungstrajektorien als graphische Elemente, insbesondere als eine Linie und/oder ein Pfeil, wiedergegeben werden. Die Fortsetzungstrajektorien können dabei insbesondere zumindest teilweise nichtlinear, beispielsweise gekrümmt, verlaufen.

Auf diese Weise wird die Korrektur des Instrumentenpfades, insbesondere für unerfahrene Anwender, deutlich vereinfacht, und Eingriffe können zeiteffizienter und unter geringerem Strahlungseinsatz durchgeführt werden. Insbesondere wird auf diese Weise ein verbessertes "Step-and-Shoot"-Verfahren bereitgestellt, bei dem die Berücksichtigung der Biegsamkeit des medizinischen Instruments und die zugeordnete Gabe von Positionierhinweisen als Workflowhinweise einen deutlichen Mehrwert liefert.

Dabei sei an dieser Stelle bereits angemerkt, dass das hier beschriebene Verfahren insbesondere ohne eine vorausgehende Planung durchgeführt werden kann. Derartiges war bei bisherigen, durch Lichtmuster geführten Eingriffen mit länglichen medizinischen Instrumenten, insbesondere Nadeln, nicht möglich. Im Rahmen der vorliegenden Erfindung kann der Benutzer zunächst ungeführt, gegebenenfalls auch unter Live-Bildgebung das medizinische Instrument eine erste Strecke in das Untersuchungsobjekt einführen, wonach Bilddaten aufgenommen werden können. Mittels der Positionierhinweise kann die Instrumententrajektorie, falls gewünscht bzw. notwendig, entsprechend korrigiert werden, um den gewünschten Zielbereich, also das Instrumentenziel, zu erreichen. Auch hier ist selbstverständlich ein mehrfaches Durchführen während des Eingriffs möglich. Alternativ kann auch eine aus dem Stand der Technik bekannte Methode zur Instrumentennavigation verwendet werden, beispielsweise eine Lasernadelführung.

Wie bereits erwähnt, sind im Rahmen der vorliegenden Erfindung zwei alternative Ausführungsformen denkbar, wie mittels des Lichtmusters und der wenigstens einen Markierung der Positionierhinweis intuitiv und zielführend für den Benutzer ausgegeben werden kann.

In einer ersten, bevorzugten Ausführungsform kann vorgesehen sein, dass das Lichtmuster einen Projektionspunkt markiert, der zur Auswahl der ausgewählten Fortsetzungstrajektorie mit einer der ausgewählten Fortsetzungstrajektorie zugeordneten Markierung in Einklang zu bringen ist, wobei die Ausgabeeinheit zur Erzeugung der Darstellung mit Markierungsinformationen, die der wenigstens einen Fortsetzungstrajektorie zugeordnet sind und die zugeordnete Markierung beschreiben, ausgebildet ist. Konkret kann vorgesehen sein, dass, um den Projektionspunkt mit der Markierung in Einklang zu bringen, ein durch die Markierung definierter Zielpunkt und der Projektionspunkt in Deckung gebracht werden. Bei mehreren Fortsetzungstrajektorien und mehreren Markierungen sind zum einen die Markierungen, zum anderen die Markierungsinformationen optisch unterscheidbar. Mit anderen Worten kann vorgesehen sein, dass die Markierungen eine optisch unterscheidbare Eigenschaft aufweisen. Die Fortsetzungstrajektorien können zur Ausgabe der Markierungsinformationen ebenso mit optisch unterscheidbaren Eigenschaften dargestellt werden, wobei insbesondere die optisch unterscheidbare Eigenschaft der Fortsetzungstrajektorien oder generell der Markierungsinformationen mit der optisch unterscheidbaren Eigenschaft jeweils einer der Markierungen paarweise korrespondieren kann. Die mehreren Markierungen können beispielsweise eine Farbcodierung und/ oder eine Schwarz-Weiß-Codierung und/oder eine Oberflächenbeschaffenheit, insbesondere eine Reflektivität und/oder Kontur, als die optisch unterscheidbare Eigenschaft aufweisen und mittels der optisch unterscheidbaren Eigenschaft können die mehreren Markierungen eindeutig identifizierbar sein. In einer konkreten Ausführungsform können bei Farbcodierung von Markierungen entsprechende Farben auch für die Fortsetzungstrajektorien in der Darstellung gewählt werden.

Der Verarbeitungseinrichtung sind die insbesondere mehreren Markierungen und ihre Lage auf dem Ausrichtungselement bekannt, so dass die Auswertungseinheit dazu ausgebildet sein kann, gezielt für Markierungen und die daraus folgenden Positionskorrekturen, umfassend eine Verbiegung des medizinischen Instruments am proximalen Anteil, die passenden Fortsetzungstrajektorien zu ermitteln, wie oben bereits dargelegt wurde.

Wie noch dargelegt werden wird, kann der Projektionspunkt bei noch nicht vorgenommener Positionskorrektur beispielsweise entlang der Längsrichtung zumindest des proximalen Anteils des Instruments, insbesondere einer Schaftachse eines Instrumentenschafts des Instruments, projiziert werden. Diese Einstellung kann durch Ansteuern der Lichtführungseinrichtung, insbesondere auf Basis der Positionierungsdaten oder aufgrund einer Planungsinformation, mittels einer Ansteuereinheit der Verarbeitungseinrichtung herbeigeführt werden. Anders gesagt kann die Verarbeitungseinrichtung die Ansteuereinheit aufweisen, die ausgebildet ist, die Lichtführungseinrichtung zur Ausgabe des Lichtmusters derart anzusteuern, dass der Projektionspunkt entlang einer aktuellen Längsrichtung wenigstens des proximalen Anteils des medizinischen Instruments, insbesondere entlang einer Schaftachse eines Instrumentenschafts, projiziert wird. Zusätzlich oder alternativ kann auch vorgesehen sein, dass die Ansteuereinheit ausgebildet ist, eine Patientenliege, auf der das Untersuchungsobjekt angeordnet ist, derart anzusteuern, dass die relative Position und Orientierung von dem medizinischen Instrument und dem Lichtmuster derart ist, dass der Projektionspunkt entlang einer aktuellen Längsrichtung wenigstens des proximalen Anteils des medizinischen Instruments, insbesondere entlang einer Schaftachse des Instrumentenschafts, projiziert wird. Zweckmäßig kann hierbei ein Mittelpunkt des Ausrichtungselements dem Projektionspunkt entsprechen, der die aktuelle Längsrichtung anzeigt.

In dieser ersten Ausführungsform kann das Ausrichtungselement als eine Art "Zielscheibe" verstanden werden, wobei zur Durchführung der Positionskorrektur der proximale Anteil des medizinischen Instruments, insbesondere an einem vorgegebenen Angriffspunkt, derart manipuliert wird, dass der Projektionspunkt mit einer zu einer ausgewählten Fortsetzungstrajektorie korrespondierenden Markierung übereinstimmt. Welche Markierung dies ist, wird durch die Markierungsinformationen in der Darstellung vermittelt.

In einer zweckmäßigen Weiterbildung der Erfindung kann vorgesehen sein, dass das Lichtmuster wenigstens eine die Orientierung der Darstellung anzeigende Orientierungslinie umfasst, insbesondere eine Bildebene einer in der Darstellung verwendeten Schicht der Bilddaten. Hierzu kann die Lichtführungseinrichtung über die Ansteuereinheit entsprechend ansteuerbar sein und/oder mit einer medizinischen Bildgebungseinrichtung, die die Bilddaten bereitstellt, registriert sein. Insbesondere kann bei einer Computertomographieeinrichtung, bei der üblicherweise axiale Schichten als Schichtbilder rekonstruiert werden, die Schichtorientierung auch fest vorgegeben sein, sodass auch die Lichtführungseinrichtung zur fest eingestellten Projektion der Orientierungslinie derart, dass diese Schichtorientierung anzeigt, ausgebildet sein kann. Eine derartige Orientierungslinie kann insbesondere durch einen Laserfächer angezeigt werden. Bei der Verwendung von Computertomographieeinrichtungen können mithin bereits vorgesehene Lichtführungseinrichtungen, die die Schichtorientierung auf dem Patienten anzeigen, auch im Rahmen der vorliegenden Erfindung zweckmäßig genutzt werden.

Dabei sei darauf hingewiesen, dass bei entsprechend ansteuerbarer Lichtführungseinrichtung mit verstellbarem Lichtfächer und/oder Laserfächer für die Orientierungslinie bei Anpassung der Darstellungsorientierung auch eine Anpassung der Orientierungslinie erfolgen kann. Dies ist insbesondere dann zweckmäßig, wenn nicht, wie häufig vorgesehen, innerhalb derselben axialen Schicht gearbeitet werden soll, sondern auch ein Wechsel dieser Schicht stattfinden kann. In diesem Fall oder auch wenn, wie oben dargelegt, die Lichtführungseinrichtung nicht ohnehin fest auf die (feste) Schichtorientierung eingestellt ist, kann die Ansteuereinheit der Verarbeitungseinrichtung ausgebildet sein, um die Lichtführungseinrichtung zur Projektion der Orientierungslinie gemäß der aktuellen Darstellungsorientierung anzusteuern.

In diesem Zusammenhang kann vorgesehen sein, dass das Lichtmuster eine weitere, insbesondere zu der Orientierungslinie senkrechte Projektionslinie umfasst. Eine derartige weitere Projektionslinie kann beispielsweise die Mitte der Bildgebungseinrichtung, insbesondere im Fall einer Computertomographieeinrichtung, anzeigen. Sie kann jedoch auch über einen entsprechenden Aktor geeignet verstellbar sein, beispielsweise um eine aktuelle Längsrichtung zumindest des proximalen Anteils des medizinischen Instruments anzuzeigen. Denkbar ist es ferner, die relative Position und Orientierung der weiteren Projektionslinie durch eine Ansteuerung der verstellbaren Patientenliege anzupassen.

In besonders vorteilhafter Weiterbildung dieser ersten Ausführungsform kann jedoch vorgesehen sein, dass das Lichtmuster zur Markierung des Projektionspunkts auf der Orientierungslinie einen, insbesondere durch die Verarbeitungseinrichtung aufgrund der Positionierungsinformation ansteuerbar einstellbaren, Orientierungspunkt umfasst. Ein derartiger Orientierungspunkt, der eine Position entlang der Orientierungslinie anzeigt, kann beispielsweise auf einfache Art und Weise durch einen Laserstrahl einer entsprechenden Laserlichtquelle der Lichtführungseinrichtung vorgegeben werden. Hierbei ist es besonders zweckmäßig, wenn die Orientierungslinie und der Orientierungspunkt unterschiedliche Farben aufweisen. Beispielsweise kann bei einer roten Orientierungslinie ein grüner Orientierungspunkt verwendet werden. Dabei ist der Orientierungspunkt durchaus als ausgedehnte, insbesondere zumindest im Wesentlichen kreisförmige Form zu verstehen, wobei ein kleines Projektionselement hier zur einfachen Umsetzung bevorzugt ist. Beispielsweise kann es sich bei dem Orientierungspunkt auch um einen kurzen Orientierungsstrich, der entlang der Orientierungslinie wandern kann, handeln. Die Position des Orientierungspunkts entlang der Orientierungslinie kann durch Ansteuerung mittels der Ansteuereinheit der Verarbeitungseinrichtung eingestellt werden. Das Vorsehen eines solchen Orientierungspunkts ist ein einfach umsetzbares, besonders zweckmäßiges Mittel vor allem dann, wenn, beispielsweise bei einer selbst verstellbaren, die Mitte der Bildgebungseinrichtung anzeigenden weiteren Projektionslinie die seitliche Feststellbarkeit der Patientenliege nicht ausreichend ist.

In einer besonders zweckmäßigen Weiterbildung kann vorgesehen sein, dass die Markierungen wenigstens eine mit der Orientierungslinie in Deckung zu bringende Ausrichtungslinie aufweisen, insbesondere derart, dass bei Verbleiben des Projektionspunkts auf der in Deckung gebrachten Ausrichtungslinie das medizinische Instrument in der in der Darstellung dargestellten Bildebene bzw. Schicht verbleibt. Dies ist insbesondere dann zweckmäßig, wenn eine axiale Schicht eines rekonstruierten Computertomographiebildes als Bilddaten verwendet wird, wie oben bereits dargelegt. Das Ausrichtungselement kann, um die Ausrichtungslinie und die Orientierungslinie in Deckung bringen zu können, an dem medizinischen Instrument drehbar angeordnet sein; denkbar ist es jedoch auch, das medizinische Instrument um seine Längsachse zu drehen.

Die Ausrichtungslinie gemeinsam mit der Orientierungslinie sorgen bei dem Benutzer für eine intuitive Orientierung, die es nicht nur vereinfacht, die Darstellung zu interpretieren, sondern auch, Markierungen korrekt dargestellten Fortsetzungstrajektorien zuzuordnen. Dies kann auch dann zweckmäßig sein, wenn nicht, wie meist üblich, in der dargestellten Schicht gearbeitet wird, sondern aus dieser heraus bewegt werden soll. Üblicherweise jedoch wird in einer bestimmten Schicht gearbeitet, um auch die weitere Bildgebung, beispielsweise bei erneuter Aufnahme von Bilddaten, zu vereinfachen.

Hierbei kann auch das Ausrichtungselement auf dieses Arbeiten in einer bestimmten Schicht hin ausgebildet sein. Beispielsweise kann vorgesehen sein, dass Fortsetzungstrajektorien zuordenbare Markierungen entlang der Ausrichtungslinie vorgesehen sind, so dass bei mit der Orientierungslinie in Deckung gebrachter Ausrichtungslinie für jede Positionskorrektur das medizinische Instrument in einer durch die Orientierungslinie markierten Schicht verbleibt. Hierbei kann das Ausrichtungselement zweckmäßigerweise in Richtung der Ausrichtungslinie länglich ausgebildet sein. Diese Ausgestaltung hat sich als besonders nützlich in Verbindung mit der Verwendung eines Projektionspunkts gezeigt, da dann auch die Ausdehnung des Lichtmusters hauptsächlich in Richtung der Orientierungslinie gegeben ist. Um eine Positionskorrektur innerhalb der Schicht, die auch der Bildebene der Darstellung entspricht, vorzunehmen, muss dann lediglich die Ausrichtungslinie mit der Orientierungslinie des Lichtmusters in Deckung gebracht werden und der Projektionspunkt auf die entsprechende Markierung entlang der Ausrichtungslinie, die gemäß der Markierungsinformationen in der Darstellung der ausgewählten Fortsetzungstrajektorie entspricht, gebracht werden.

Konkrete Ausführungsbeispiele im Rahmen der ersten Ausführungsform können ferner vorsehen, dass
- die Markierungen wenigstens einen Teil eines die wenigstens eine Ausrichtungslinie umfassenden Fadenkreuzes bilden, dessen Mitte vor Vornahme der Positionskorrektur mit dem Projektionspunkt übereinstimmt, und dessen Ringe, insbesondere in unterschiedlichen Farben, jeweiligen Fortsetzungstrajektorien zugeordnet sind, und/oder
- die Markierungen ein, insbesondere matrixartiges, Koordinatenraster bilden, wobei zumindest den Fortsetzungstrajektorien zuordenbaren Markierungen Koordinaten zugeordnet sind.

Ein Fadenkreuz hat den Vorteil, dass schnell und einfach die Orientierungslinie mit einer der (dann zwei zueinander senkrecht stehenden) Ausrichtungslinien in Deckung gebracht werden kann, insbesondere, wenn ohnehin in einer bestimmten Schicht dauerhaft gearbeitet werden soll. Beispielsweise können die Markierungen dann farbige Kreise des Fadenkreuzes bilden, wobei der Projektionspunkt dann in eine gewollte Richtung auf der gewählten, mit der Orientierungslinie in Deckung gebrachten Ausrichtungslinie zu einer gewünschten Markierung einer gewünschten, durch die Markierungsinformationen angegebenen Farbe zur Positionskorrektur bewegt werden kann. Dabei sei an dieser Stelle noch generell angemerkt, dass beim Arbeiten innerhalb einer Schicht, die auch die Bildebene der Darstellung bildet, zweckmäßig ohnehin jeweils zwei Fortsetzungstrajektorien einer Farbe, insbesondere der Farbe eines Kreises des Fadenkreuzes, bestimmt werden können, nämlich eine für jede Korrekturrichtung innerhalb der Schicht. Durch die intuitive Orientierung mittels der Orientierungslinie ist es für den Benutzer problemlos möglich, die entsprechenden Richtungen der Korrektur innerhalb der Schicht korrekt zuzuordnen.

Bei der Verwendung von Markierungen in einem matrixartigen Koordinatenraster, beispielsweise Markierungspunkten bzw. Markierungskreisen in einer regelmäßigen Matrixanordnung, können beispielsweise Koordinatenangaben als Markierungsinformationen verwendet werden, beispielsweise derart, dass für eine B3-Fortsetzungstrajektorie der Projektionspunkt entsprechend auf die Markierung im Koordinatenraster bei B3 zu bewegen ist. Aber auch, wenn der Projektionspunkt nicht im Zentrum angeordnet werden kann, das bedeutet, nicht zwangsläufig immer die gleiche Richtung, beispielsweise die Längsrichtung zumindest des proximalen Anteils des medizinischen Instruments, anzeigt, ist ein Koordinatenraster äußerst nützlich, denn dann kann beispielsweise eine relative Zuordnung im Koordinatenraster erfolgen, beispielsweise der Art "eins nach rechts, drei nach unten".

In der alternativen, zweiten Ausführungsform wird ein anderer Ansatz gewählt, in dem das Lichtmuster, insbesondere der Projektionspunkt, angibt, wohin die (gegebenenfalls dann eine) Markierung zu bewegen ist. Das bedeutet, in dieser Ausführungsform ist vorgesehen, dass das Ausrichtungselement nur eine einen Ausrichtpunkt definierende Markierung aufweist und die Verarbeitungseinrichtung ausgebildet ist, die Lichtführungseinrichtung zur Markierung eines einer Fortsetzungstrajektorie zugeordneten Projektionspunkts auf der Projektionsfläche anzusteuern, der zur Auswahl der zugeordneten Fortsetzungstrajektorie mit dem Ausrichtpunkt in Einklang, also in Deckung, zu bringen ist. Eine derartige Ausgestaltung ist insbesondere dann zweckmäßig, wenn eine bestmöglich zu einem Zielbereich bzw. Instrumentenziel im Eingriffsbereich führende Fortsetzungstrajektorie ermittelt wird und diese möglichst genau eingehalten werden soll. Dann kann der Projektionspunkt anzeigen, wohin der durch die wenigstens eine Markierung definierte Ausrichtpunkt zu bewegen ist, um die Positionskorrektur zum Erreichen dieser ausgewählten Fortsetzungstrajektorie durchzuführen. Denkbar ist es grundsätzlich jedoch auch, dass mehrere, unterscheidbare, unterschiedlichen Fortsetzungstrajektorien zugeordnete Projektionspunkte als Teil des Lichtmusters projizierbar sind. Anders gesagt können Projektionspunkte unterscheidbare optische Eigenschaften aufweisen, die durch die Markierungsinformationen beschrieben werden bzw. deren Markierungsinformationen in ihren unterscheidbaren optischen Eigenschaften mit den unterscheidbaren optischen Eigenschaften der Projektionspunkte korrespondieren, sodass eine Auswahl einer Fortsetzungstrajektorie gemäß der Darstellung durch Bewegen des Ausrichtpunkts auf den entsprechenden Projektionspunkt erfolgen kann.

In der zweiten Ausführungsform wird zweckmäßigerweise die Ansteuereinheit als Teil der Verarbeitungseinrichtung benötigt, um die Lichtführungseinrichtung und/oder eine Patientenliege geeignet anzusteuern, dass der wenigstens eine Projektionspunkt die jeweilige Fortsetzungstrajektorie korrekt anzeigt.

Konkret kann beispielsweise vorgesehen sein, dass Laserlichtquellen zugeordnete Aktoren, beispielsweise zur Verstellung von in den Raum projizierten Laserstrahlen bzw. gekreuzten Laserebenen, derart angesteuert werden, dass diejenige Verschiebung des proximalen Endes des medizinischen Instruments durch den Projektionspunkt angezeigt wird, welche als Positionskorrektur realisiert werden muss, ob eine bestimmte, beispielsweise durch Benutzereingabe und/oder automatisch, ausgewählte Fortsetzungstrajektorie zu realisieren. Mit anderen Worten wird das Lichtmuster verschoben und zeigt als neuen Projektionspunkt den gewünschten Verbiegungsgrad des medizinischen Instruments an.

Somit ist in der zweiten Ausführungsform ein einfach gestaltetes Ausrichtungselement ausreichend und Fortsetzungstrajektorien, insbesondere am besten zu einem Instrumentenziel führende Fortsetzungstrajektorien, können hochgenau, intuitiv und einfach eingestellt werden.

Dabei sei dieser Stelle noch angemerkt, dass für beide Ausführungsformen eine gezielte Auswahl einer von mehreren Fortsetzungstrajektorien durch Benutzereingabe umgesetzt denkbar ist. Beispielsweise kann der Benutzer in der zweiten Ausführungsform eine Fortsetzungstrajektorie aus mehreren Vorschlägen auswählen, für die dann der Projektionspunkt die notwendige Positionskorrektur anzeigt. Insbesondere kann bei der ersten Ausführungsform dann vorgesehen sein, dass die Markierungsinformationen erst in die Darstellung eingeblendet werden, wenn diese Auswahl erfolgt ist, beispielsweise im Hinblick auf ein Koordinatenraster, für das dann eine Ausgabe für eine relative Verschiebung im Koordinatenraster erfolgen kann.

In einer zweckmäßigen Weiterbildung der vorliegenden Erfindung kann vorgesehen sein, dass der proximale Anteil einen Angriffspunkt zur manuellen Vornahme der Positionskorrektur aufweist, wobei die Auswertungseinheit zur Berücksichtigung der Lage des Angriffspunktes bei der Ermittlung der wenigstens einen Fortsetzungstrajektorie ausgebildet ist. Dabei befindet sich der vorgesehene Angriffspunkt bevorzugt am proximalen Ende des Instruments und/oder benachbart dem Ausrichtungselement und/oder an dem Ausrichtungselement, wobei jedoch auch andere Angriffspunkte denkbar sind. Je nachdem, wo sich der Angriffspunkt befindet, mithin wo der Benutzer das medizinische Instrument zur Positionskorrektur greift, treten, insbesondere hinsichtlich der Verbiegung, andere Formen am proximalen Anteil auf, die erfindungsgemäß unter Kenntnis des Angriffspunkts optimal berücksichtigt und in die Ermittlung der Fortsetzungstrajektorien einbezogen werden können. Je weiter außen der Angriffspunkt liegt, desto mehr kann von den Biegungseigenschaften des Instruments profitiert werden. Bei Ausführungsformen, in denen unterschiedliche Angriffspunkte vorgesehen bzw. nutzbar sind, kann beispielsweise der aktuell genutzte Angriffspunkt aus einer Benutzereingabe hergeleitet werden.

In einer bevorzugten Ausgestaltung kann die Eingriffsvorrichtung eine Bildgebungseinrichtung zur Aufnahme wenigstens eines Teils der Bilddaten aufweisen. Derartige Eingriffs-Arbeitsplätze, die auch bereits eine Bildgebungseinrichtung, insbesondere zur Überwachung des Eingriffs, aufweisen, wurden im Stand der Technik grundsätzlich bereits vorgeschlagen. Hierbei kann die Verarbeitungseinrichtung auch eine Aufnahmeeinheit zur Ansteuerung der Bildgebungseinrichtung zur Aufnahme der Bilddaten aufweisen. Besonders bevorzugt ist die Nutzung einer Computertomographieeinrichtung, da dies für insbesondere geführte Eingriffe mit länglichen medizinischen Instrumenten, insbesondere Nadeln, bereits grundsätzlich bekannt ist und Vorgehensweisen, insbesondere bei Durchführung einer Planung, übernommen werden können. Eine Computertomographievorrichtung liefert, insbesondere für eine den Eingriffsbereich umfassende axiale Schicht, auf einfache und schnelle Art und Weise dreidimensionale Bilddaten, die insbesondere auch das Instrument deutlich zeigen und somit gut auswertbar sind. Neben einer Computertomographieeinrichtung sind alternativ oder zusätzlich auch andere Bildgebungseinrichtungen, insbesondere Röntgeneinrichtungen, denkbar. So kann die Bildgebungseinrichtung beispielsweise auch eine C-Bogen-Röntgeneinrichtung sein, die einen C-Bogen aufweist, an dem sich gegenüberliegend ein Röntgenstrahler und ein Röntgendetektor angeordnet sind. Durch die Bewegungsfreiheitsgrade des C-Bogens können verschiedene Projektionsgeometrien/Aufnahmegeometrien eingestellt werden. Grundsätzlich sind selbstverständlich auch weitere Bildgebungsmodalitäten denkbar.

Hinsichtlich einer C-Bogen-Röntgeneinrichtung oder einer sonstigen, unterschiedliche Aufnahmegeometrien erlaubenden Röntgeneinrichtung sei noch angemerkt, dass hier meist eine fluoroskopische Überwachung stattfindet. Dabei kann die Aufnahmegeometrie, insbesondere die Angulation des C-Bogens, geändert werden, um den Instrumentenpfads in allen Raumrichtungen erkennen zu können. Typische Beispiele für Eingriffe, die unter Fluoroskopiebeobachtung durchgeführt werden, sind Knochenbiopsie, Spinalfusion und Vertebroplastie. Zur Ermittlung der Bilddaten, aus denen Schlussfolgerungen zur Positionierung des Instruments und/oder der Anatomie gezogen werden sollen, kann in solchen Fällen vorgesehen sein, Fluoroskopiebilder des medizinischen Instruments aus wenigstens zwei Projektionsrichtungen aufzunehmen, um die gewünschten dreidimensionalen Informationen zu erhalten. Denkbar ist es jedoch auch, eine C-Bogen-CT-Akquisition vorzunehmen.

Generell kann es zweckmäßig sein, wenn die Lichtführungseinrichtung an der Bildgebungseinrichtung fest verbaut und/oder in diese integriert ist. Für Computertomographieeinrichtungen ist dies bei der Durchführung von minimalinvasiven Eingriffen mit länglichen Instrumenten, insbesondere Nadeln, bereits bekannt, um ein Instrumentenführungssystem umzusetzen und/oder einem Benutzer eine optimale Orientierung zu bieten, beispielsweise durch Anzeige der Orientierung der axialen Schicht (insbesondere mittels der Orientierungslinie) und/ oder der Mitte der Bildgebungseinrichtung. Die Lichtführungseinrichtung kann hier beispielsweise an der Gantry angeordnet sein. Auch bei einem C-Bogen ist derartiges grundsätzlich möglich, beispielsweise, indem nach der Aufnahme der Bilddaten bei unterschiedlichen Angulationen wieder in die sogenannte "Bull's Eye View" gefahren wird, in der die Lichtführungseinrichtung bzw. das Lichtmuster dem Orientierungspunkt entlang der Längsachse zumindest des proximalen Anteils des Instruments ausgerichtet ist. Beispielsweise kann die Lichtführungseinrichtung, insbesondere Laserführungseinrichtung, in diesem Fall am Röntgendetektor angeordnet sein, wie dies beispielsweise in der Veröffentlichung "Improved Laser Needle Guidance" von Dr. Alois Regensburger et al., Siemens AG 2019, beschrieben ist.

Wie bereits erwähnt, kann die Auswertungseinheit zur Ermittlung der Positionierungsdaten wenigstens teilweise aus den Bilddaten ausgebildet sein. Auf diese Weise sind keinerlei zusätzliche Positionierungsdaten erforderlich und/oder diese können plausibilisiert werden. Insbesondere bei der Röntgenbildgebung ist eine solche Detektion des Instruments sowie die Ermittlung der Positionierungsdaten leicht mit gängigen Algorithmen möglich, da das medizinische Instrument ein Hochkontrastobjekt darstellt.

Besonders zweckmäßig ist es, wenn die Auswertungseinheit zur Registrierung der Bilddaten mit bereitgestellten Vorab-Bilddaten des Untersuchungsobjekts ausgebildet ist. Insbesondere im Fall von Fluoroskopiebildern wird bei der Röntgenbildgebung teilweise die Anatomie, welche ja auch von Interesse ist, weniger deutlich abgebildet, während sie in Vorab-Bilddaten leicht erkannt bzw. segmentiert werden kann. Auch allgemein kann mithin in einer zweckmäßigen Weiterbildung eine Registrierung zu einem solchen Vorab-Bilddatensatz, beispielsweise einem dreidimensionalen Computertomographie-Bilddatensatz/oder Magnetresonanz-Bilddatensatz, vorgenommen werden, wobei in dem Vorab-Bilddatensatz die Anatomie, beispielsweise unterschiedliche Gewebearten, bereits segmentiert sein kann bzw. segmentierten Bereichen mechanische Eigenschaften zugeordnet sein können. Bevorzugt werden jedoch den aktuellen Zustand der Anatomie im Eingriffsbereich beschreibende Anatomieinformationen direkt aus den Bilddaten ermittelt, um beispielsweise aufgetretene Verschiebungen und/oder sonstige Bewegungen nachvollziehen zu können. Derartige Anatomieinformationen können auch verwendet werden, um den Vorab-Bilddatensatz, beispielsweise hinsichtlich Gewebegrenzen, zu aktualisieren. Dies kann auch durch die Registrierung selbst geschehen, wenn eine elastische Registrierung hinsichtlich der Anatomie im Eingriffsbereich durchgeführt wird. Dann, wenn die Bilddaten die Anatomie auch hinreichend deutlich zeigen, führt eine elastische Registrierung letztlich unmittelbar zu einer Information über aufgetretene Veränderungen.

Insbesondere kann es sich bei den Bilddaten, die die aktuelle Situation mit teilweise eingeführtem medizinischem Instrument zeigen, allgemein gesprochen, um solche eines Kontrollscans handeln. Aus den Bilddaten kann im Rahmen der Auswertung eine Ermittlung der Positionierungsdaten erfolgen, wie bereits dargelegt, wobei durch Auswertung der Bilddaten (insbesondere gemeinsam mit starr oder bevorzugt elastisch hierzu registrierten Vorab-Bilddaten) bevorzugt durch übliche Bildauswertungsmethoden, insbesondere umfassend Segmentierung und/oder Positionsbestimmung und/oder Detektion von Strukturen, folgende Auswertungsinformationen erhalten werden können:
- Positionierungsinformationen (Lage des Instruments, Form des Instruments (beispielsweise Centerline entlang des Instruments und/oder funktionale Approximation der Form des Instruments) sowie Orte des distalen sowie, falls sichtbar, des proximalen Endes des Instruments),
- Eintrittspunkt des Instruments in das Untersuchungsobjekt sowie Anteile des Instruments, welche sich innerhalb und außerhalb des Untersuchungsobjekts befinden (falls der proximale Anteil des Instruments, der sich außerhalb des Untersuchungsobjekts befindet, nicht oder nicht vollständig in den Bilddaten enthalten ist, kann unter Annahme einer kräftefreien, also nicht aktiv ausgelenkten Lage des Instruments der Verlauf bis zum proximalen Ende extrapoliert werden, wozu die Eigenschaftsdaten zweckmäßig auch weitere Informationen zum medizinischen Instrument enthalten können, insbesondere zur Länge und dergleichen).
- bevorzugt Anatomieinformationen, insbesondere umfassend die aktuelle Lage von anatomischen Strukturen, beispielsweise von Organen und deren Organgrenzen, insbesondere umfassend den Zielbereich. Des Weiteren kann eine Elastizität und/oder Beweglichkeit der anatomischen Strukturen bekannt sein oder angenommen werden.

Auch die Lage einer ursprünglich geplanten Instrumententrajektorie (Planungstrajektorie) kann in den Bilddaten nachverfolgt werden, insbesondere bei Registrierung mit Vorab-Bilddaten, auf denen die Planung erfolgte.

Basierend auf den vorliegenden Informationen kann dann eine Simulation und/oder Modellberechnung dahingehend erfolgen, wie sich eine Positionskorrektur, umfassend eine Verbiegung des Instruments, am hinteren (proximalen) Ende des Instruments auf die Lage und Form des Instruments innerhalb des Patienten sowie die Trajektorie am vorderen (distalen) Ende des Instruments (bei weiterem Vorschub) auswirken würde. Dabei werden auch die Eigenschaftsdaten, die die Biegbarkeit des medizinischen Instruments beschreiben (insbesondere sowohl im proximalen Anteil als auch distal), berücksichtigt. Hierbei umfassen die Eigenschaftsdaten insbesondere mechanische und/ oder elastische Parameter des Instruments. Auch für die Anatomie können, wie erwähnt, die mechanischen Eigenschaften bekannt sein oder angenommen werden.

Diesbezüglich kann zweckmäßigerweise vorgesehen sein, dass die Auswertungseinheit zur Ermittlung der Eigenschaftsdaten aus einer bereitgestellten Instrumenteninformation ausgebildet ist. Die Instrumenteninformation kann dabei beispielsweise ein Modell und/oder eine Art und/oder einen Typ des Instruments beschreiben. Beispielsweise können die Eigenschaftsdaten der Instrumenteninformation in einer Datenbank und/oder einer Look-Up-Tabelle (LUT) zugeordnet sein. Grundsätzlich ist es jedoch auch denkbar, Standardannahmen für die Eigenschaftsdaten zu treffen.

Vorzugsweise kann vorgesehen sein, dass die Auswertungseinheit zusätzlich zu der wenigstens einen Fortsetzungstrajektorie auch zur Ermittlung einer sich ohne Positionskorrektur ergebenden Zukunftstrajektorie und die Ausgabeeinheit zur Anzeige der Zukunftstrajektorie in der Darstellung ausgebildet ist. Auf diese Weise erhält der Benutzer auch eine Information darüber, wie sich der Instrumentenpfad weiterentwickelt, wenn er keine Positionskorrektur vornimmt. Die Fortsetzungstrajektorien können insbesondere intuitiv und einfach mit der Zukunftstrajektorie verglichen und bezüglich des Erreichens des Zielbereichs bewertet werden.

Ferner ist es zweckmäßig, wenn die Auswertungseinheit zur Ermittlung einer Empfehlung für eine Vorschubstrecke und die Ausgabeeinheit zu deren Ausgabe ausgebildet ist. Beispielsweise kann hierbei eine Verlässlichkeit der Vorausberechnung der Fortsetzungstrajektorien ermittelt werden, wobei bis zu einem bestimmten Schwellwert der Verlässlichkeit der Vorausberechnung ein Vorschub empfohlen werden kann. Nach einem Vorschub um die empfohlene Vorschubstrecke, welche beispielsweise in Zentimetern angegeben werden kann, kann dann zweckmäßigerweise eine erneute Aufnahme von Bilddaten, mithin ein erneuter Kontrollscan, erfolgen und es können neue Fortsetzungstrajektorien für Positionskorrekturen ermittelt werden.

Neben der Eingriffsvorrichtung betrifft die Erfindung auch ein computerimplementiertes Verfahren zur Ermittlung und Ausgabe eines Positionierhinweises für ein medizinisches, längliches Instrument, das für einen Eingriff in einen Eingriffsbereich eines Untersuchungsobjekts teilweise einführbar ist, einen proximalen, außerhalb des Untersuchungsobjekts anzuordnenden Anteil zur manuellen Handhabung des medizinischen Instruments aufweist und wenigstens teilweise biegbar ist, wobei ein an dem Instrument angeordnetes und/oder in dieses integriertes Ausrichtungselement, das eine Projektionsfläche mit wenigstens einer Markierung, die wenigstens einen Punkt markiert, verwendet wird, wobei das Verfahren bei bereits teilweise in das Untersuchungsobjekt eingeführtem Instrument folgende Schritte aufweist:
- Bereitstellen von Bilddaten des Untersuchungsobjekts, die den Eingriffsbereich darstellen, über eine Schnittstelle einer Verarbeitungseinrichtung und Ermittlung von Positionierungsdaten, die die aktuelle Position und Lage des medizinischen Instruments beschreiben,
- mittels einer Auswertungseinheit der Verarbeitungseinrichtung, Ermittlung wenigstens einer prädiktiven, insbesondere nichtlinearen, Fortsetzungstrajektorie aus den Bilddaten und den Positionierungsdaten unter Verwendung von die Biegbarkeit des medizinischen Instruments beschreibenden Eigenschaftsdaten,
- Ausgabe einer auf den Bilddaten basierenden Darstellung des Eingriffsbereichs und der wenigstens einen Fortsetzungstrajektorie mittels einer Ausgabeeinheit der Verarbeitungseinrichtung an einer Darstellungseinrichtung; und
- insbesondere mittels einer Ansteuereinheit der Verarbeitungseinrichtung, Betrieb einer Lichtführungseinrichtung zur Projektion wenigstens eines Lichtmusters auf die Projektionsfläche derart, dass durch Zusammenschau wenigstens einer der wenigstens einen Markierung und des Lichtmusters der Positionierhinweis zur manuellen Positionskorrektur an dem proximalen Anteil des medizinischen Instruments zum Erreichen einer auszuwählenden der wenigstens einen Fortsetzungstrajektorie bei weiterem Einschub des medizinischen Instruments entsteht.

Sämtliche Ausführungen bezüglich der erfindungsgemäßen Eingriffsvorrichtung lassen sich analog auf das erfindungsgemäße Verfahren, welches auch als Hinweisverfahren bezeichnet werden kann, übertragen, und umgekehrt, sodass die bereits genannten Vorteile erhalten werden können. Insbesondere erfolgt die Ermittlung der Positionierungsdaten bevorzugt durch die Auswertungseinheit unter Verwendung der Bilddaten. Das Verfahren kann auch den Schritt der Aufnahme der Bilddaten, gesteuert durch eine Aufnahmeeinheit, umfassen.

Der Positionierhinweis kann dabei als ein Workflowhinweis verstanden werden, sodass eine Unterstützung bzw. Entscheidungshilfe für den Benutzer entsteht, die nach Positionierung des medizinischen Instruments, insbesondere im Rahmen eines Kontrollscans, der die Bilddaten liefert, bereitgestellt wird. Somit ist darauf hinzuweisen, dass selbst bei Anwendung im Rahmen eines Eingriffs an einem Patienten im Rahmen des erfindungsgemäßen Hinweisverfahren keinerlei Führung, Vorschub oder sonstige Maßnahme an dem medizinischen Instrument im Untersuchungsobjekt erfolgt.

Auch für das Verfahren sind, wie oben für die Eingriffsvorrichtung bereits dargelegt, zwei grundlegende, alternative Ausführungsformen denkbar. Zum einen kann das Lichtmuster einen Projektionspunkt markieren, der zur Auswahl der ausgewählten Fortsetzungstrajektorie mit einer der ausgewählten Fortsetzungstrajektorie zugeordneten Markierung in Einklang zu bringen ist, wobei die Darstellung mit der wenigstens einen Fortsetzungstrajektorie zugeordneten, die zugeordnete Markierung beschreibenden Markierungsinformationen erzeugt wird. Zum anderen kann vorgesehen sein, dass das Ausrichtungselement nur eine einen Ausrichtpunkt definierende Markierung aufweist und die Verarbeitungseinrichtung die Lichtführungseinrichtung zur Markierung eines einer Fortsetzungstrajektorie zugeordneten Projektionspunkts auf der Projektionsfläche ansteuert, wobei der Projektionspunkt zur Auswahl der zugeordneten Fortsetzungstrajektorie mit dem Ausrichtpunkt in Einklang zu bringen ist.

Das Hinweisverfahren lässt sich in ein Verfahren zur Behandlung und/oder Untersuchung eines Untersuchungsobjekts mittels eines medizinischen, längliches Instruments, das für einen Eingriff in einen Eingriffsbereich eines Untersuchungsobjekts teilweise eingeführt wird, einen proximalen, außerhalb des Untersuchungsobjekts anzuordnenden Anteil zur manuellen Handhabung des medizinischen Instruments aufweist und wenigstens teilweise biegbar ist, integrieren, wobei zur Ermittlung und Ausgabe eines Positionierhinweises nach Einführen des Instruments in das Untersuchungsobjekt
- ein an dem Instrument angeordnetes und/oder in dieses integriertes Ausrichtungselement, das eine Projektionsfläche mit wenigstens einer Markierung, die wenigstens einen Punkt markiert, verwendet wird,
- Bilddaten des Untersuchungsobjekts, die den Eingriffsbereich darstellen, über eine Schnittstelle einer Verarbeitungseinrichtung bereitgestellt werden und Positionierungsdaten, die die aktuelle Position und Lage des medizinischen Instruments beschreiben, ermittelt werden,
- mittels einer Auswertungseinheit der Verarbeitungseinrichtung, wenigstens eine prädiktiven, insbesondere nichtlineare, Fortsetzungstrajektorie aus den Bilddaten und den Positionierungsdaten unter Verwendung von die Biegbarkeit des medizinischen Instruments beschreibenden Eigenschaftsdaten ermittelt wird,
- eine auf den Bilddaten basierende Darstellung des Eingriffsbereichs und der wenigstens einen Fortsetzungstrajektorie mittels einer Ausgabeeinheit der Verarbeitungseinrichtung auf einer Darstellungseinrichtung ausgegeben wird; und
- insbesondere mittels einer Ansteuereinheit der Verarbeitungseinrichtung, eine Lichtführungseinrichtung zur Projektion wenigstens eines Lichtmusters auf die Projektionsfläche derart betrieben wird, dass durch Zusammenschau wenigstens einer der wenigstens einen Markierung und des Lichtmusters der Positionierhinweis zur manuellen Positionskorrektur an dem proximalen Anteil des medizinischen Instruments zum Erreichen einer auszuwählenden der wenigstens einen Fortsetzungstrajektorie bei weiterem Einschub des medizinischen Instruments entsteht,
wonach der Positionierungshinweis durch eine den Eingriff durchführende Person an dem proximalen Anteil des Instruments, insbesondere an dem Angriffspunkt, manuell umgesetzt wird und das Instrument weiter in das Untersuchungsobjekt eingeschoben wird.

Das Verfahren kann bevorzugt iterativ durchgeführt werden, das bedeutet, nach einer bestimmten, insbesondere empfohlenen, Vorschubstrecke des Instruments können neue Bilddaten aufgenommen/bereitgestellt werden und auf deren Basis ein weiterer Positionierhinweis mit dem Hinweisverfahren generiert und ausgegeben werden.

Ein erfindungsgemäßes Computerprogramm ist direkt in ein Speichermittel einer Verarbeitungseinrichtung einer Eingriffsvorrichtung ladbar und weist Programmmittel auf, die bei Ausführung des Computerprogramms die Verarbeitungseinrichtung veranlassen, die Schritte eines erfindungsgemäßen Hinweisverfahrens durchzuführen. Das Computerprogramm kann auf einem elektronisch lesbaren Datenträger gespeichert sein, welcher mithin darauf gespeicherte Steuerinformation umfasst, die wenigstens ein erfindungsgemäßes Computerprogramm umfassen und derart ausgestaltet sind, dass bei Verwendung des Datenträgers in einer Verarbeitungseinrichtung einer Eingriffsvorrichtung diese ausgestaltet wird, ein erfindungsgemäßes Hinweisverfahren auszuführen.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: einen Ablaufplan eines allgemeinen Ausführungsbeispiels des Verfahrens,
- Fig. 2: eine Prinzipskizze wesentlicher Komponenten einer erfindungsgemäßen Eingriffsvorrichtung,
- Fig. 3: den funktionalen Aufbau der Verarbeitungseinrichtung der Eingriffsvorrichtung,
- Fig. 4: eine schematische Detailansicht eines medizinischen Instruments für eine erste Ausführungsform der Erfindung,
- Fig. 5: eine Lichtführungseinrichtung für ein erstes Ausführungsbeispiel der ersten Ausführungsform,
- Fig. 6: ein Ausrichtungselement für das erste Ausführungsbeispiel,
- Fig. 7: eine Illustration der Hinweisgabe in einem ersten Zustand von Instrument und Ausrichtungselement,
- Fig. 8: eine Illustration des ersten Zustands auf der Projektionsfläche,
- Fig. 9: eine Illustration der Hinweisgabe in einem zweiten Zustand von Instrument und Ausrichtungselement,
- Fig. 10: eine Illustration des zweiten Zustands auf der Projektionsfläche,
- Fig. 11: eine Illustration der Hinweisgabe in einem dritten Zustand von Instrument und Ausrichtungselement,
- Fig. 12: eine Illustration des dritten Zustands auf der Projektionsfläche,
- Fig. 13: eine dem ersten Ausführungsbeispiel zugehörige Darstellung zur Ausgabe an einer Darstellungseinrichtung,
- Fig. 14: ein Ausrichtungselement für ein zweites Ausführungsbeispiel der ersten Ausführungsform,
- Fig. 15: eine Illustration der Hinweisgabe in einem ersten Zustand von Instrument und Ausrichtungselement,
- Fig. 16: eine Illustration des ersten Zustands auf der Projektionsfläche,
- Fig. 17: eine Illustration der Hinweisgabe in einem zweiten Zustand von Instrument und Ausrichtungselement,
- Fig. 18: eine Illustration des zweiten Zustands auf der Projektionsfläche,
- Fig. 19: eine Illustration der Hinweisgabe in einem dritten Zustand von Instrument und Ausrichtungselement,
- Fig. 20: eine Illustration des dritten Zustands auf der Projektionsfläche,
- Fig. 21: ein Ausrichtungselement für ein drittes Ausführungsbeispiel der ersten Ausführungsform,
- Fig. 22: eine schematische Teilansicht eines Instruments mit integriertem Ausrichtungselement für die zweite Ausführungsform der Erfindung, und
- Fig. 23: eine Illustration zur Erläuterung eines Ausführungsbeispiels der zweiten Ausführungsform.

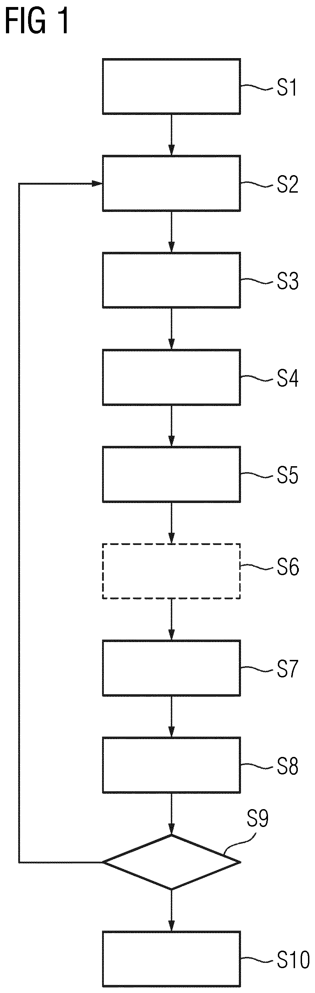

Fig. 1 zeigt einen Ablaufplan eines allgemeinen Ausführungsbeispiels der Führung eines medizinischen Instruments, hier eine Nadel, zu einem Zielbereich, welche ein erfindungsgemäßes Hinweisverfahren in den Schritten S2 bis S7 umfasst.

In einem vor Beginn des Hinweisverfahrens durchgeführten Schritt S1 findet ein Einschub des medizinischen Instruments in das Untersuchungsobjekt, beispielsweise einen Patienten oder ein Untersuchungsphantom, statt. Dies kann gemäß einer vorab geplanten Instrumententrajektorie, also einer Planungstrajektorie, erfolgen, die, wie grundsätzlich bekannt, auf Planungs-Bilddaten (die später erwähnten Vorab-Bilddaten entsprechen können) geplant wurde. Die Planungstrajektorie kann hierbei durch Laserführung mittels einer Lichtführungseinrichtung, die auch im folgenden Hinweisverfahren verwendet wird, eingestellt werden und das medizinische Instrument wird gemäß dieser Planungstrajektorie ein Stück weit in das Untersuchungsobjekt eingeführt. Durch diesen Vorgang, aber auch andere (gewollte oder ungewollte) Bewegungsvorgänge im Eingriffsbereich, in dem auch ein Instrumentenziel (Zielbereich) liegt, kann es jedoch zu Veränderungen kommen, sodass mit der Planungstrajektorie nicht zwangsläufig der Zielbereich erreicht wird.

Eine Planung ist mit der hier vorgestellten Vorgehensweise allerdings nicht zwangsläufig nötig, das bedeutet, ein Benutzer, beispielsweise ein Arzt, kann zunächst ungeführt bzw. unter Live-Bildgebung das medizinische Instrument um eine erste Strecke vorschieben und dennoch aufgrund des nun folgenden Hinweisverfahren verlässlich den Zielbereich erreichen.

In einem ersten Schritt des erfindungsgemäßen Hinweisverfahrens, Schritt S2, werden bei bereits teilweise in das Untersuchungsobjekt eingeführtem medizinischen Instrument mittels einer Bildgebungseinrichtung gesteuert durch eine Aufnahmeeinheit einer Verarbeitungseinrichtung der Eingriffsvorrichtung, mit der der Eingriff durchgeführt wird, als Kontrollscan Bilddaten des Eingriffsbereichs mit dem Instrument aufgenommen. Hierbei kann es sich um eine dreidimensionale Aufnahme, beispielsweise einen Computertomographie-Scan, handeln, es ist jedoch auch denkbar, ein oder mehrere zweidimensionale Bilder als Bilddaten aufzunehmen, beispielsweise bei Verwendung einer C-Bogen-Röntgeneinrichtung zweidimensionale Röntgenbilder (insbesondere Fluoroskopiebilder) aus unterschiedlichen Projektionsrichtungen (Angulationen), also mit unterschiedlichen Aufnahmegeometrien. Zur Aufnahme kann auch, falls notwendig, die Rekonstruktion von Bildern, beispielsweise eines axialen Schichtbilds, dass den Eingriffsbereich abbildet, bei der Computertomographie, gehören.

Liegen, insbesondere dreidimensionale, Vorab-Bilddaten des Eingriffsbereichs vor, deren Bildinformationen für die folgende Auswertung nützlich sind, kann auch eine starre oder bevorzugt elastische Registrierung der Bilddaten zu den Vorab-Bilddaten erfolgen, beispielsweise dem Planungs-Bilddatensatz. Bei einer elastischen Registrierung enthalten die Registrierungs-Transformationen bereits Informationen zu gegebenenfalls aufgetretenen Verformungen der Anatomie im Eingriffsbereich; bei der starren Registrierung lassen sich lediglich einfache translatorische Bewegungen folgern.

In einem Schritt S3 findet dann unter Verwendung üblicher Auswertungsmethoden eine Auswertung der Bilddaten, insbesondere gemeinsam mit den hierzu registrierten Vorab-Bilddaten, in einer Auswertungseinheit der Verarbeitungseinrichtung statt. Beispielsweise können Segmentierungsalgorithmen, Positionsbestimmungsalgorithmen, Klassifizierungsalgorithmen, Detektionsalgorithmen und dergleichen eingesetzt werden.

Im Rahmen der Auswertung werden zunächst Positionierungsinformationen hinsichtlich des länglichen, wenigstens teilweise elastisch biegbaren, medizinischen Instruments ermittelt. Diese können beispielsweise umfassen
- die Lage des medinischen Instruments,
- die Form des (hier eine gewisse Biegbarkeit aufweisenden) medizinischen Instruments (beispielsweise als Centerline und/oder als funktionale Beschreibung der Form),
- Positionen des distalen und des proximalen Endes des Instruments, wobei dann, wenn der außerhalb des Untersuchungsobjekts befindliche, proximale Anteil des Instruments in den Bilddaten nicht oder nur teilweise sichtbar ist, aufgrund bekannter Eigenschaften des Instruments (insbesondere dessen Länge) bei Annahme von Kräftefreiheit, das proximale Ende dennoch abgeschätzt werden kann, und/oder
- den Einstichpunkt (Eintrittspunkt) des medizinischen Instruments in den Körper des Untersuchungsobjekts sowie Anteile des medizinischen Instruments, die sich innerhalb (distaler Anteil) und außerhalb (proximale Anteil) des Untersuchungsobjekts befinden.

Im Schritt S3 können durch Auswertung der Bilddaten ferner Anatomieinformationen ermittelt werden, insbesondere die Lage des Zielbereichs (Instrumentenziels), die Lage von anatomischen Strukturen und/oder Begrenzungen der organischen Strukturen (beispielsweise von Organen) und/oder die Lage der, falls vorhanden, Planungstrajektorie in der Anatomie.

Die Bestimmung der Anatomieinformation kann wenigstens teilweise unter Berücksichtigung der registrierten Vorab-Bilddaten erfolgen, insbesondere, wenn in den Vorab-Bilddaten diese anatomischen Strukturen bereits segmentiert und/oder markiert sind.

In einem Schritt S4 wird dann eine Simulation und/oder eine Modellberechnung durchgeführt, wie sich eine Positionskorrektur am proximalen Anteil des Instruments, insbesondere am proximalen Ende des Instruments, umfassend eine Verbiegung des Instruments, auf die Lage und die Form des Instruments innerhalb des Untersuchungsobjekts, also des distalen Anteils, auswirkt. Insbesondere wird dabei vorausberechnet, was bei Vorliegen der Positionskorrektur und Vorschub des medizinischen Instruments als Fortsetzungstrajektorie resultieren würde. Zusätzlich zu diesen Positionskorrekturen und den zugeordneten Fortsetzungstrajektorien wird auch eine ohne Positionskorrektur resultierende Zukunftstrajektorie des medizinischen Instruments vorausberechnet, welche sich ergibt, wenn das medizinische Instrument ohne Positionskorrektur am proximalen Ende vorgeschoben wird. All diese Berechnungen erfolgen unter Berücksichtigung der Biegsamkeit des medizinischen Instruments, mithin von Eigenschaftsdaten, die diese elastische Biegbarkeit des medizinischen Instruments beschreiben. Derartige Eigenschaftsdaten können beispielsweise mechanische Parameter und/oder Elastizitätsparameter umfassen. Während die Eigenschaftsdaten auf einer Standardannahme beruhen können, ist es bevorzugt, diese, beispielsweise aus einer Datenbank und/oder einer Look-Up-Tabelle in Abhängigkeit von einer bereitgestellten, beispielsweise aus einer Benutzereingabe gefolgerten, Instrumenteninformation abzurufen. Die Instrumenteninformation kann beispielsweise ein Modell und/oder eine Art und/oder einen Typ des medizinischen Instruments beschreiben.

Für welche Positionskorrekturen und auf welche Art die wenigstens eine Fortsetzungstrajektorie ermittelt wird, hängt davon ab, welcher konkrete Ansatz gewählt wird. Ist an dem medizinischen Instrument ein Ausrichtungselement mit einer Projektionsfläche vorgesehen, auf der mehrere unterschiedliche, in der Auswertungseinheit bekannte Positionskorrekturen beschreibende Markierungen vorgesehen sind, können für all diese Markierungen und die zugeordneten Positionskorrekturen Fortsetzungstrajektorien ermittelt werden. Wird aber auf der Projektionsfläche, beispielsweise durch ein Lichtmuster der mittels einer Ansteuereinheit ansteuerbaren Lichtführungseinrichtung, eine bestimmte Positionskorrektur vorgeschlagen, kann auch diese beispielsweise in einem Optimierungsverfahren derart ermittelt werden, dass der Zielbereich möglichst genau erreicht wird.

Schließlich kann, für jede der Fortsetzungstrajektorien einzeln oder aber für alle Fortsetzungstrajektorien insgesamt, im Schritt S4 eine empfohlene Vorschubstrecke bis zum nächsten Kontrollscan ermittelt werden, beispielsweise in Abhängigkeit eines die Verlässlichkeit der Vorausberechnung über diese Strecke für die Fortsetzungstrajektorie bzw. Fortsetzungstrajektorien anzeigenden Verlässlichkeitswerts.

Bei all diesen Auswertungen wird im Übrigen auch die Lage des Angriffspunktes zur Vornahme der Positionskorrektur am proximalen Anteil des Instruments berücksichtigt. Bevorzugt ist hierbei eine Lage des Angriffspunktes am proximalen Ende des medizinischen Instruments. Je nach Lage des Angriffspunktes kann es zu anderen Formen, insbesondere Verbiegungen, des proximalen Anteils kommen, welche entsprechend berücksichtigt werden.

In einem Schritt S5 wird mittels einer Ausgabeeinheit der Verarbeitungseinrichtung eine Darstellung erzeugt und mittels einer Darstellungseinrichtung, beispielsweise eines von einer das medizinische Instrument manuell betätigenden Person gut einsehbaren Monitors, ausgegeben. Die Darstellung basiert auf den Bilddaten, beispielsweise dem bereits erwähnten axialen Schichtbild, weist mithin eine bestimmte Darstellungsorientierung (als Bildebene der Bilddaten in der Darstellung) auf. Durch die Bilddaten zeigt die Darstellung auch das medizinische Instrument und die Anatomie im Eingriffsbereich, wobei sowohl das medizinische Instrument als auch anatomische Strukturen, insbesondere wenn diese segmentiert sind und ihre Begrenzungen bekannt sind, zur besseren Erkennbarkeit hervorgehoben und/oder kontrastiert sein können. Entsprechende Verfahren zur Erzeugung geeigneter Darstellungen sind bekannt. Die Darstellung enthält in jedem Fall auch, beispielsweise durch Überlagerung, graphische Elemente, die die wenigstens eine Fortsetzungstrajektorie und die Zukunftstrajektorie ortsgenau einblenden. Wird eine empfohlene Vorschubstrecke ermittelt, kann auch diese ausgegeben werden. Hierauf wird im Hinblick auf die verschiedenen Ausführungsbeispiele im Folgenden noch genauer eingegangen werden.

In einem optionalen Schritt S6 kann eine Auswahl wenigstens einer der Fortsetzungstrajektorien aufgrund einer Benutzereingabe erfolgen. Dies ist insbesondere dann zweckmäßig, wenn mehrere Fortsetzungstrajektorien ermittelt wurden und eine Auswahlmöglichkeit durch Identifikation einer geeigneten Markierung bzw. eines geeigneten unterscheidbaren Projektionspunkts, worauf ebenso im Weiteren noch eingegangen werden wird, nicht vorgesehen ist. Die Auswahl kann insbesondere durch Beurteilung der Fortsetzungstrajektorien in der Darstellung erfolgen.

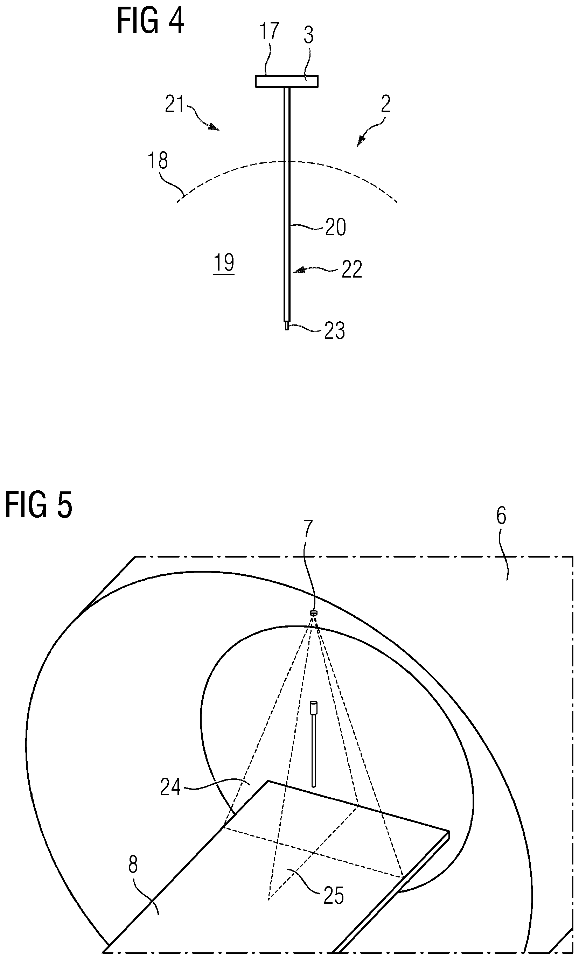

In einem Schritt S7 wird schließlich ein Positionierhinweis zur Einstellung einer entsprechenden manuellen Positionskorrektur an dem proximalen Anteil des medizinischen Instruments zum Erreichen einer auszuwählenden der wenigstens einen Fortsetzungstrajektorie bei weiterem Einschub des medizinischen Instruments ausgegeben, wobei der Positionierhinweis durch Zusammenschau eines von der Lichtführungseinrichtung ausgesandten Lichtmusters auf der Projektionsfläche, der wenigstens einen Markierung auf der Projektionsfläche sowie gegebenenfalls der Darstellung entsteht. Nachdem hierfür unterschiedliche Ansätze im erfindungsgemäßen Hinweisverfahren denkbar sind, sollen diese im Hinblick auf die Fig. 4 bis 23 näher erläutert werden.

Damit ist das Hinweisverfahren abgeschlossen und in einem auf dieses Hinweisverfahren folgenden Schritt kann die den Eingriff durchführende Person, also der Benutzer, dem Positionierhinweis folgend die Positionskorrektur vornehmen, mithin die Voraussetzungen für die ausgewählte Fortsetzungstrajektorie herstellen, und sodann durch manuellen Vorschub das medizinische Instrument in Richtung des Instrumentenziels weiter vorschieben, beispielsweise um die empfohlene Vorschubstrecke.

In einem Schritt S9 wird dann überprüft, ob das Instrumentenziel, insbesondere also der Zielbereich, erreicht wurde und mit dem Grund des Eingriffs fortgefahren werden kann, mithin im Schritt S10 der eigentliche Eingriffsgrund ausgeführt werden kann. Ansonsten wird das Hinweisverfahren, Schritte S2 bis S7, umfassend einen neuen Kontrollscan, erneut ausgeführt, um den Benutzer beim Erreichen des Instrumentenziels unterstützen. Es sei angemerkt, dass der Kontrollscan selbstverständlich auch bereits als Grundlage des Schrittes S9 durchgeführt werden kann.

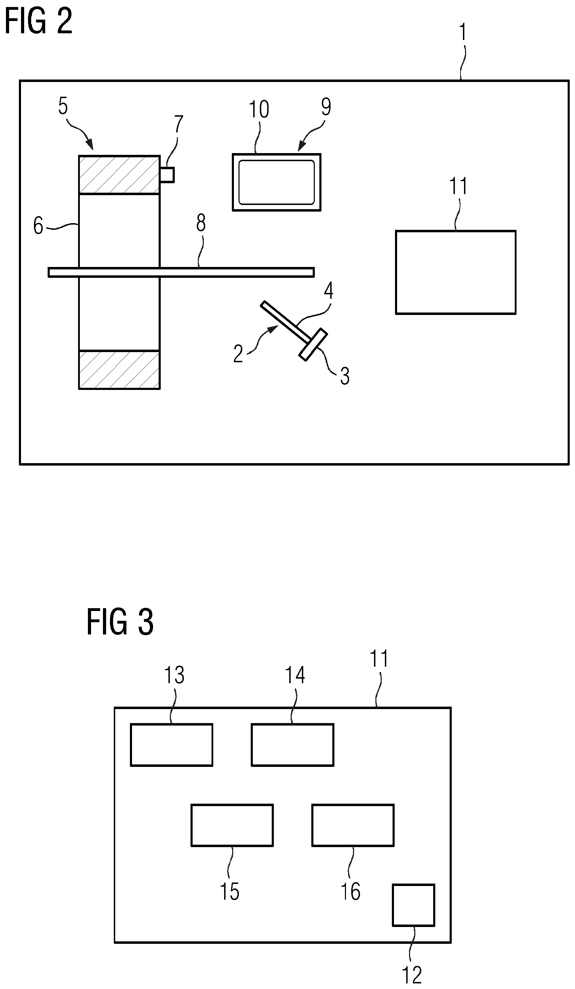

Fig. 2 zeigt in Form einer Prinzipskizze wesentliche Komponenten einer erfindungsgemäßen Eingriffsvorrichtung 1. Die Eingriffsvorrichtung 1 umfasst zunächst das medizinische Instrument 2, an dem das Ausrichtungselement 3 befestigt ist bzw. in das das Ausrichtungselement 3 integriert ist. Das medizinische Instrument 2 ist als Nadel 4 ausgebildet. Die Bildgebungseinrichtung 5 ist vorliegend eine Computertomographieeinrichtung mit einer Gantry 6, an der vorliegend die Lichtführungseinrichtung 7 angeordnet ist. Die Lichtführungseinrichtung 7 kann ein oder mehrere Laserlichtquellen zur Erzeugung von Laserfächern und/oder Laserstrahlen aufweisen, sodass bei entsprechender Ausrichtung auf die Projektionsfläche das Lichtmuster entsteht.

In die Gantry 6 kann das Untersuchungsobjekt mittels einer Patientenliege 8 eingefahren werden.

Die Darstellungseinrichtung 9 ist vorliegend als Monitor 10 ausgebildet.

Der Betrieb der Eingriffsvorrichtung 1, insbesondere zur Durchführung des Hinweisverfahrens, wird von der Verarbeitungseinrichtung 11 gesteuert, deren funktionaler Aufbau in Fig. 3 genauer erläutert ist. Neben einem Speichermittel 12 umfasst die Verarbeitungseinrichtung 11 zunächst eine Aufnahmeeinheit 13 zur Ansteuerung der Bildgebungseinrichtung 5 zur Aufnahme der Bilddaten sowie gegebenenfalls zu deren Registrierung mit Vorab-Bilddaten und/oder zur Rekonstruktion von Volumina und/oder Schichtbildern, vorliegend insbesondere axialen Computertomographie-Schichtbildern. Mit anderen Worten ist die Aufnahmeeinheit 13 zur Durchführung des Schrittes S2 ausgebildet. Die Auswertungseinheit 14 ist zur Auswertung der Bilddaten und zur Ermittlung der Fortsetzungstrajektorien sowie gegebenenfalls der Zukunftstrajektorie und der empfohlenen Vorschubstrecke gemäß den Schritten S3 und S4 ausgebildet. Die Ausgabeeinheit 15 dient zur Erzeugung und Ausgabe der Darstellung gemäß dem Schritt S5, während die Ansteuereinheit 16 zur Ansteuerung der Lichtführungseinrichtung 7, insbesondere im Rahmen des Schrittes S7, ausgebildet ist. Selbstverständlich sind auch weitere Funktionseinheiten bzw. Sub-Funktionseinheiten denkbar.

Fig. 4 zeigt schematisch eine Ansicht des Instruments 2, an dem das Ausrichtungselement 3, vorliegend am proximalen Ende, angebracht ist. Auf seiner Oberseite bildet das Ausrichtungselement 3 die Projektionsfläche 17. Nimmt man die gestrichelte Linie 18 als Begrenzung des Untersuchungsobjekts 19 an, so weist das medizinische Instrument 2, welches als Nadel 4 vorliegend einen länglichen, elastisch biegbaren Instrumentenschaft 20 aufweist, einen proximalen Anteil 21 und einen distalen Anteil 22 mit dem distalen Ende 23 auf, welches das Instrumentenziel, also den Zielbereich, erreichen soll.

Fig. 5 zeigt beispielhaft eine mögliche Ausgestaltung der Lichtführungseinrichtung 7 an der Gantry 6. Die Lichtführungseinrichtung 7 weist vorliegend Laserlichtquellen zur Erzeugung zweier Laserfächer 24, 25 auf, wobei beispielsweise der Laserfächer 24 zumindest im Grundbetrieb der Bildgebungseinrichtung 5 die Lage bzw. Orientierung der rekonstruierten axialen Schicht auf dem Untersuchungsobjekt in Form einer Orientierungslinie anzeigen kann. Mittels des Laserfächers 25 kann eine Mitte der Gantry 6 bzw. der Bildgebungseinrichtung 5 angezeigt werden. Den Laserlichtquellen können Aktoren zugeordnet sein, um die Laserfächer 24 und 25 zu verstellen; die relative Position kann auch durch Ansteuerung der Patientenliege 8 mittels der Ansteuereinheit 16 angepasst werden.

Selbstverständlich sind auch eine Vielzahl anderer Ausgestaltungen der Lichtführungseinrichtung 7 denkbar, um für die im Folgenden näher diskutierten Ausgestaltungen geeignete Lichtmuster auf die Projektionsfläche 17 projizieren zu können. Insbesondere kann auch eine Laserlichtquelle zur Projektion eines Laserstrahls und somit eines Orientierungspunkts zusätzlich zu der Orientierungslinie vorgesehen sein.

Eine erste Ausführungsform, zu der nun einige Ausführungsbeispiele erläutert werden, sieht vor, dass das Lichtmuster einen Projektionspunkt markiert, der zur Auswahl der ausgewählten Fortsetzungstrajektorie mit einer der ausgewählten Fortsetzungstrajektorie zugeordneten Markierung in Einklang zu bringen ist. Hierzu ist die Ausgabeeinheit 15 ausgebildet, die Darstellung mit der wenigstens einen Fortsetzungstrajektorie zugeordneten, die zugeordnete markierungsbeschreibenden Markierungsinformationen zu erzeugen. Dabei wird zweckmäßigerweise in den im Folgenden dargestellten Ausführungsbeispielen der ersten Ausführungsform innerhalb der Schicht des axialen Schichtbilds, dass auch die Grundlage der Darstellung bildet, gearbeitet.

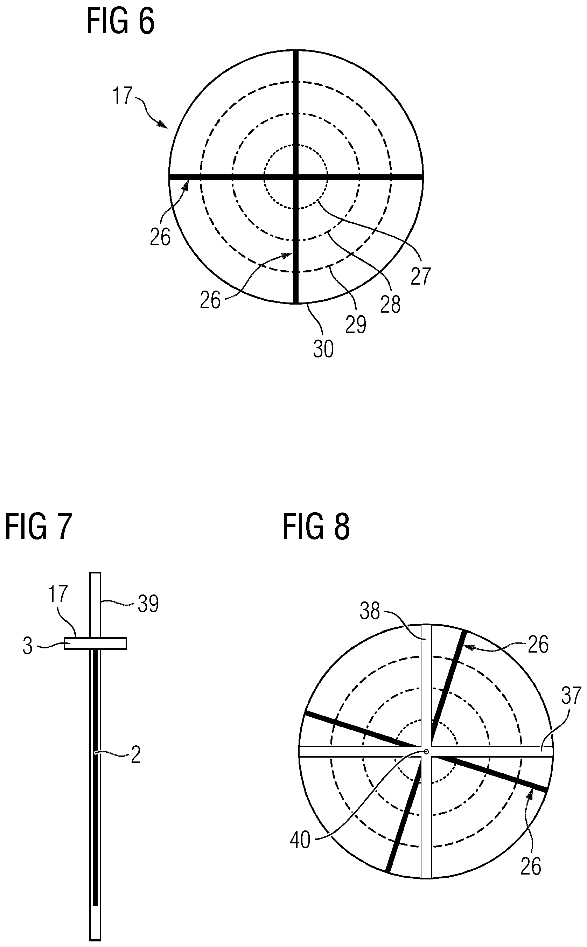

Fig. 6 zeigt für ein erstes Ausführungsbeispiel eine mögliche Ausgestaltung der Projektionsfläche 17 des Ausrichtungselements 3. Diese weist Markierungen nach Art eines Fadenkreuzes auf, wobei das Kreuz durch zwei senkrecht zueinander verlaufende Ausrichtungslinien 26 gebildet wird. Um den Mittelpunkt des Kreuzes verlaufen konzentrische Kreise bzw. Ringe 27, 28 und 29, die in unterschiedlichen Farben (als unterschiedliche optische Eigenschaft) aufgebracht sind, beispielsweise der Kreis 27 in Grün, der Kreis 28 in Blau und der Kreis 29 in Violett. Der Kreis 30 stellt die äußere Berandung dar. Ersichtlich kreuzen die Kreise 27 bis 29 die Ausrichtungslinien 26 mehrmals.

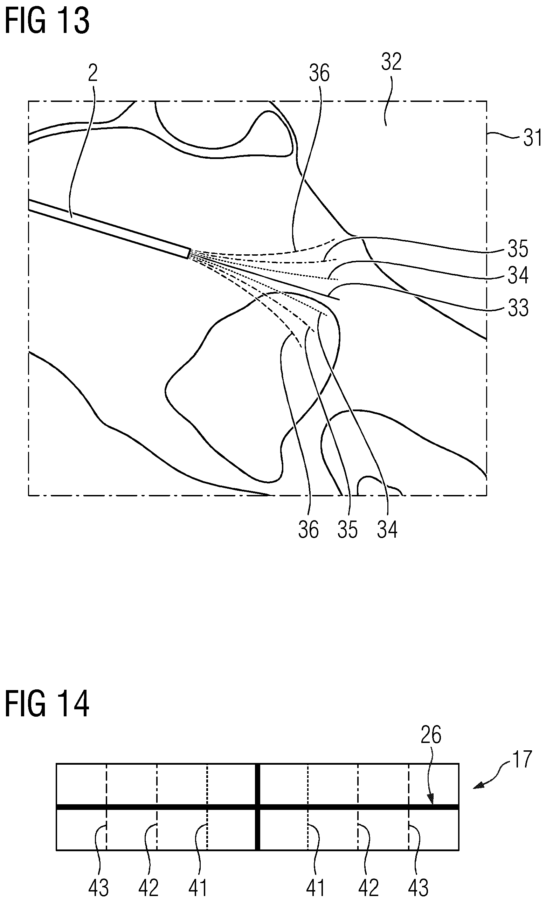

Die Fig. 7 bis 12 erläutern nun die Nutzung dieses Ausrichtungselements 3 zur Ausgabe des Positionierungshinweises, wobei Fig. 13 eine mögliche Darstellung 31 auf der Darstellungseinrichtung 9 wiedergibt. Ersichtlich basiert die Darstellung 31 auf Bilddaten 32, in denen die Anatomie sowie das medizinische Instrument 2 teilweise zu sehen sind. Das mittlere graphische Element 33 zeigt die Zukunftstrajektorie, die diesem in beide Richtungen benachbarten graphischen Elemente 34, 35, 36 zeigen Fortsetzungstrajektorien an. Hierbei ist das graphische Element 33 in einer Farbe, beispielsweise Orange, gehalten, die bei den Kreisen 27, 28 und 29 nicht vorkommt, während die unterscheidbare optische Eigenschaft der graphischen Elemente 34, 35 und 36 jeweils der der Kreise 27, 28 und 29 entspricht. Mit anderen Worten sind die graphischen Elemente 34 grün, die graphischen Elemente 35 blau und die graphischen Elemente 36 violett. Die graphischen Elemente 34, 35 und 36 zeigen also nicht nur den Verlauf der Fortsetzungstrajektorien an, sondern enthalten auch Markierungsinformationen (die Farbe), um entsprechenden Markierungen auf der Projektionsfläche 17 des Ausrichtungselements 3 zugeordnet zu werden.

Dies ermöglicht - mit der Richtung, in der die entsprechende Fortsetzungstrajektorie von der Zukunftstrajektorie abweicht - eine eindeutige Zuordnung zu Markierungen, wenn in der Schicht des axialen Schichtbilds verblieben werden soll.

Fig. 7 und Fig. 8 stellen nun eine erste Situation dar, in der das Lichtmuster, welches vorliegend, vergleiche Fig. 8, durch eine Orientierungslinie 37 und eine hierzu senkrechte Projektionslinie 38 gebildet wird, ebenso ein Kreuz bildet, welches jedoch nicht mit dem Kreuz der Ausrichtungslinien 26 des Fadenkreuzes übereinstimmt. Die Orientierungslinie 37 zeigt die Darstellungsorientierung, also die Orientierung der Bildebene des axialen Schichtbilds, an und kann im Kontext der Bildgebungseinrichtung 5 und der Anordnung des Untersuchungsobjekts 19 auf der Patientenliege 8 vom Benutzer leicht identifiziert werden. Durch Ansteuerung der Lichtführungseinrichtung 7 und/oder der Patientenliege 8 stimmt die Richtung des Kreuzungsstrahls 39, vergleiche Fig. 7, mit der Längsrichtung zumindest des proximalen Anteils 21 des Instruments 2 überein, sodass sich der Kreuzungspunkt des Lichtmusters aus der Orientierungslinie 37 und der Projektionslinie 38, der einen Projektionspunkt 40 darstellt, und der Kreuzungspunkt der Ausrichtungslinien 26 entsprechen.

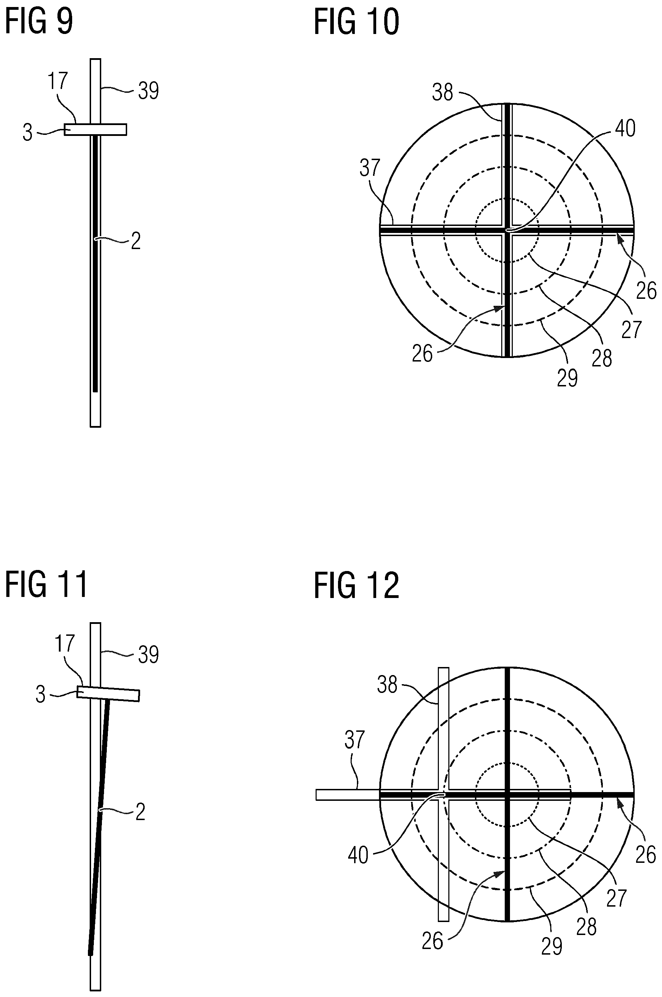

Der Benutzer dreht nun, wie durch die Fig. 9 und 10 dargestellt, das Ausrichtungselement 2 derart, dass, vergleiche Fig. 10, eine der Ausrichtungslinien 26 auf der Orientierungslinie 27 zu liegen kommt, wobei die andere Ausrichtungslinie 26 dann auf der Projektionslinie 38 liegt. Nun markieren die Schnittpunkte der Kreise 27, 28 und 29 mit der Orientierungslinie 37 bzw. der entsprechenden Ausrichtungslinie 26 Zielpunkte derart, dass dann, wenn der Projektionspunkt 40 mit dem entsprechenden Zielpunkt in Übereinstimmung gebracht wird, sich die Positionskorrektur einstellt, die zu der entsprechend farbcodierten Fortsetzungstrajektorie in der entsprechenden Richtung führt.

Denn die Ermittlung der Fortsetzungstrajektorien ist auf Basis genau dieser, durch die Zielpunkte gegebenen Positionskorrekturen in der Schicht erfolgt. Da die Lage der Markierungen, hier konkret der Kreise 27, 28 und 29, bekannt ist, sind auch die sich in der Schicht ergebenden Positionskorrekturen bekannt und im Schritt S4 können die entsprechenden Fortsetzungstrajektorien, selbstverständlich unter Berücksichtigung des Angriffspunkts, ermittelt werden. Dabei sind dem Benutzer die entsprechenden Richtungen und ihre Auswirkungen genau wie die Tatsache, welche von den Linien 37, 38 die Orientierungslinie 37 ist, intuitiv klar und deutlich bewusst, da das Untersuchungsobjekt 19 entsprechend vor ihm liegt und sich die Darstellung auch auf die Schicht bezieht.

Wie in den Fig. 11 und 12 dargestellt ist, hat der Benutzer in diesem ersten Ausführungsbeispiel die Fortsetzungstrajektorie gemäß dem unteren graphischen Element 35 (blau) ausgewählt, da sie am ehesten zum Zielbereich führt, und führt daher eine Positionskorrektur an am proximalen Ende des Instruments 2 liegenden Angriffspunkt derart durch, dass der Projektionspunkt 40, wie in Fig. 12 gezeigt, auf dem linken Schnittpunkt des blauen Kreises 28 mit der Ausrichtungslinie 26, die mit der Orientierungslinie 37 in Deckung gebracht wurde, zu liegen kommt. Erfolgt nun ein Vorschub, stellt sich die durch das untere graphische Element 35 dargestellte Fortsetzungstrajektorie ein. Hierbei verbleibt das medizinische Instrument 2 in der axialen Schicht, die durch das Schichtbild der Bilddaten 32, das der Darstellung 31 zugrunde liegt, dargestellt wird, sodass für weitere Kontrollscans sowie für die Orientierungslinie 37 keine Veränderungen erforderlich sind.

Insbesondere dann, wenn, wie dargestellt, immer in einer fest vorgegebenen Schicht gearbeitet werden soll, kann auch eine Ausgestaltung der Projektionsfläche 17 des Ausrichtungselements 3, wie sie für das zweite Ausführungsbeispiel in Fig. 14 dargestellt ist, verwendet werden. Hier ist die Projektionsfläche 17 länglich in der Richtung der (hier nur einen) Ausrichtungslinie 26 gehalten und die Kreise 27, 28, 29 sind auf zu der Ausrichtungslinie 26 senkrechte Striche 41 (grün), 42 (blau) und 43 (violett) reduziert.

In dem zweiten Ausführungsbeispiel reichen beispielhaft die Einstellungsmöglichkeiten für den zweiten Laserfächer 25 bzw. die Patientenliege 8 nicht aus, um den Projektionspunkt 40 sinnvoll in die Mitte der Projektionsfläche 17 zu liegen, sodass die Lichtführungseinrichtung zusätzlich zu der Orientierungslinie 37, die weiterhin die Darstellungsorientierung, also die Orientierung der axialen Schicht, anzeigt, durch eine Laserlichtquelle zur Ausgabe eines Laserstrahls 44 einen Orientierungspunkt 45 auf die Projektionsfläche 17 projiziert, um das Lichtmuster zu bilden und den Projektionspunkt 40 zu definieren. Der Laserstrahl 44 und der Laserfächer 24 können unterschiedliche Farben aufweisen.

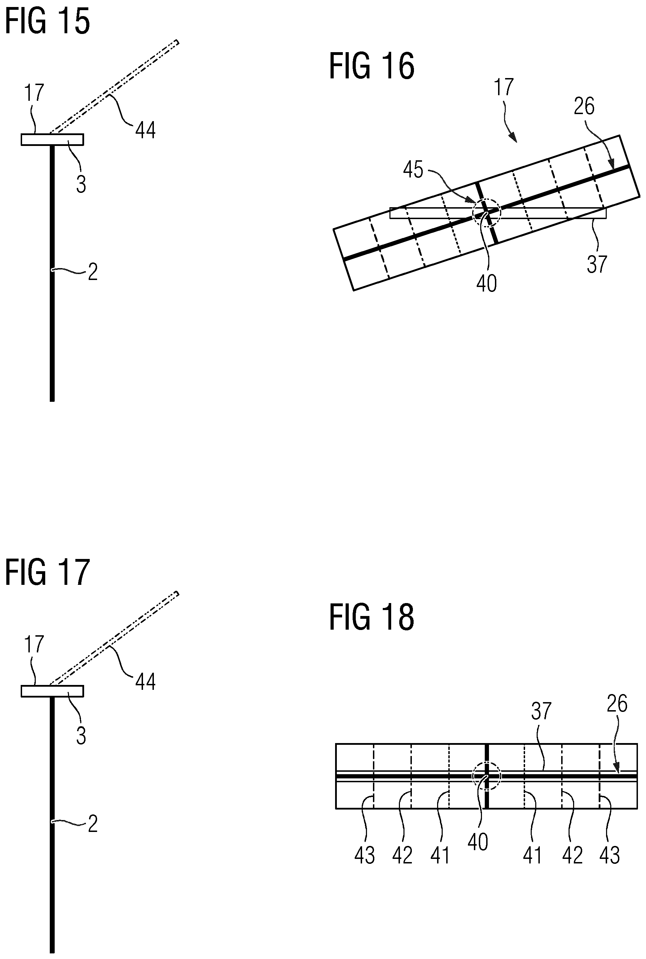

Die Fig. 15 und 16 zeigen wiederum den Anfangszustand (erste Situation), in der die Orientierungslinie 37 und die Ausrichtungslinie 26 nicht übereinstimmen, aber der Projektionspunkt 40 bereits auf dem zentralen Kreuzungspunkt der Markierungen liegt. In den Fig. 17 und 18 (zweite Situation) ist das Ausrichtungselement 3 so gedreht, dass die Orientierungslinie 37 und die Ausrichtungslinie 26 übereinanderliegen. Die Schnittpunkte der Striche 41, 42 und 43 mit der Ausrichtungslinie 26 geben analog zu den Fig. 9 und 10 nun die Zielpunkte an, auf die der Projektionspunkt 40 zur Auswahl der entsprechend in der Darstellung 31 als graphisches Element 34, 35 bzw. 36 gezeigten Fortsetzungstrajektorie zu bewegen ist.

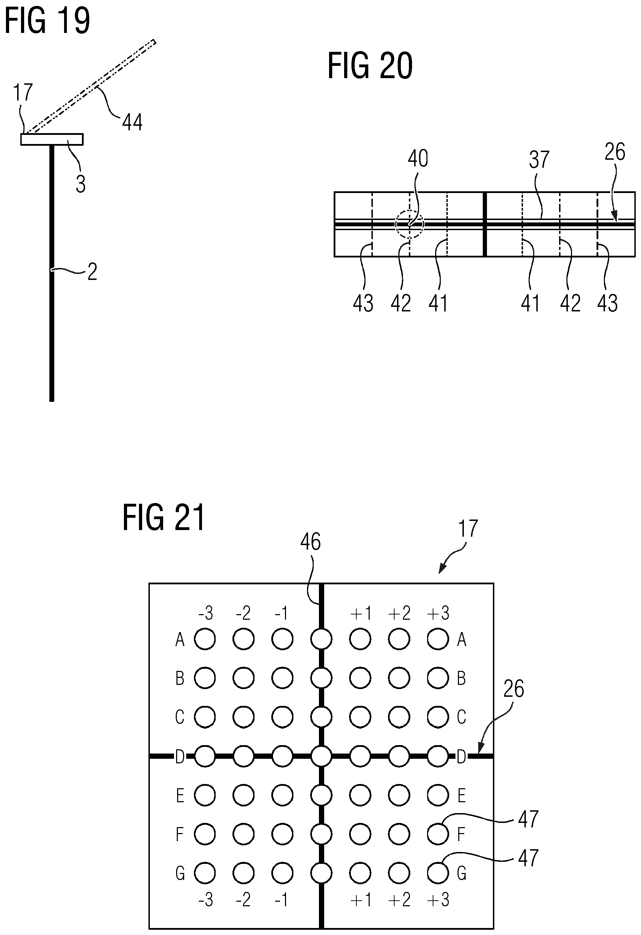

In der dritten Situation gemäß den Fig. 19 und 20 wurde wiederum die dem unteren graphischen Element 35 zugeordnete Fortsetzungstrajektorie durch entsprechende Positionskorrektur ausgewählt, da der Projektionspunkt 40 sich nun auf dem durch den linken Strich 42 (blau) definierten Zielpunkt befindet.

Fig. 21 zeigt ein drittes Ausführungsbeispiel für die erste Ausführungsform, in dem neben einem durch eine Ausrichtungslinie 26 und eine weitere Linie 46 gebildeten Kreuz Punkte 47 als matrixartig angeordnete Markierungen eines Koordinatenrasters mit entsprechenden Beschriftungen untergebracht sind. Hier können beispielsweise den graphischen Elementen 34, 35, 36 als Markierungsinformationen in der Darstellung 31 Koordinaten der entsprechenden Punkte 47 als Zielpunkte zugeordnet werden. Auch relative Angaben sind möglich, wenn beispielsweise der Projektionspunkt 40 nicht am Kreuzungspunkt der Ausrichtungslinie 26 mit der weiteren Linie 46 platziert werden kann.

In der zweiten Ausführungsform ist wenigstens eine Markierung, die einen Ausrichtpunkt anzeigt, auf der Projektionsfläche 17 ausreichend, denn die Ansteuereinheit 16 steuert hier die Lichtführungseinrichtung 7 zur Markierung eines einer Fortsetzungstrajektorie zugeordneten Projektionspunkts auf der Projektionsfläche 17 derart an, das zur Auswahl der zugeordneten Fortsetzungstrajektorie, also zur Einstellung der nötigen Positionskorrektur, dieser Projektionspunkt mit dem Ausrichtpunkt in Einklang zu bringen ist.

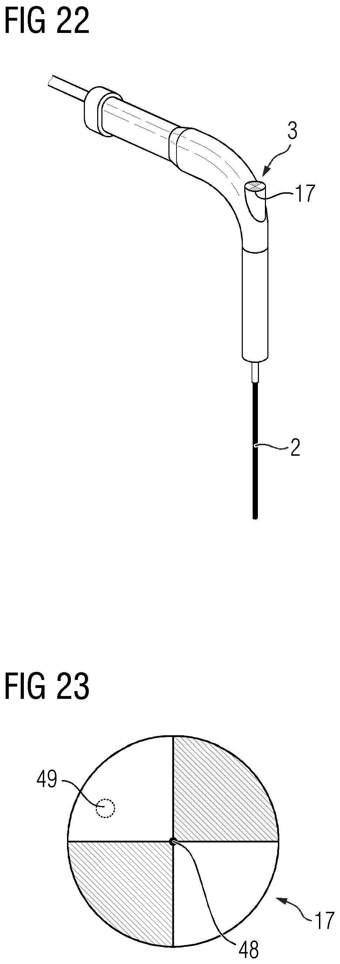

Nachdem in diesem Fall einfacher gestaltete Markierungen denkbar sind, zeigt Fig. 22 ein Ausführungsbeispiel, in dem das Ausrichtungselement 3 in das Instrument 2, nämlich eine proximale Endfläche als Projektionsfläche 17, integriert ist.

Fig. 23 zeigt diese Markierung genauer, die zentral einen (der Längsrichtung zumindest des proximalen Anteils 21 ohne Positionskorrektur entsprechenden) Ausrichtpunkt 48 definiert. Der Projektionspunkt 49 als Lichtmuster oder Teil des Lichtmusters befindet sich ersichtlich versetzt dazu. Wird nun am Angriffspunkt das Instrument 2 so in seiner Position korrigiert, dass der Ausrichtpunkt 48 und der Projektionspunkt 49 zusammenfallen, ist die Positionskorrektur für das Erreichen der dem Projektionspunkt 49 zugeordneten Fortsetzungstrajektorie vorgenommen und diese kann durch Vorschub erreicht werden.

Im Fall eines Projektionspunkts 49 können die Markierungsinformationen in der Darstellung 31 die zugeordnete Fortsetzungstrajektorie einzeln hervorheben, während bei mehreren Projektionspunkten 49 auch eine Zuordnung über unterscheidbare optische Eigenschaften erfolgen kann.

Insbesondere ist bei einem Ausführungsbeispiel der zweiten Ausführungsform, bei der nur ein Projektionspunkt 49 verwendet wird, die Nutzung des optionalen Schrittes S6 sinnvoll, um die gewünschte Fortsetzungstrajektorie beispielsweise bereits in der Darstellung 31 auszuwählen und sodann den passenden Projektionspunkt 49 zur Ausgabe des Positionierhinweises projiziert zu bekommen.

Möglich ist es in diesem Zusammenhang jedoch auch, dass eine optimal zum Erreichen des Instrumentenziels geeignete Fortsetzungstrajektorie automatisch bestimmt bzw. ausgewählt wird, in der Darstellung 31 angezeigt wird und ein geeigneter Projektionspunkt 49 mittels der Führungseinrichtung 7 projiziert wird.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

Unabhängig vom grammatikalischen Geschlecht eines bestimmten Begriffes sind Personen mit männlicher, weiblicher oder anderer Geschlechteridentität mit umfasst.

## Patentansprüche

1. Medizinische Eingriffsvorrichtung (1), aufweisend
- ein medizinisches, längliches Instrument (2), das für einen Eingriff in einen Eingriffsbereich eines Untersuchungsobjekts (19) teilweise einführbar ist, einen proximalen, außerhalb des Untersuchungsobjekts (19) anzuordnenden Anteil (21) zur manuellen Handhabung des medizinischen Instruments (2) aufweist und wenigstens teilweise biegbar ist,
- ein an dem Instrument (2) angeordnetes und/oder in dieses integriertes Ausrichtungselement (3), das eine Projektionsfläche (17) mit wenigstens einer Markierung, die wenigstens einen Punkt markiert, aufweist,
- eine Darstellungseinrichtung (9),
**dadurch gekennzeichnet, dass** die medizinische Eingriffsvorrichtung aufweist:
- eine Verarbeitungseinrichtung (11), welche aufweist:
- eine Auswertungseinheit (14), welche dazu ausgebildet ist, aus den Eingriffsbereich darstellenden Bilddaten (32) des Untersuchungsobjekts (19) und die aktuelle Position und Lage des medizinischen Instruments (2) beschreibenden Positionierungsdaten unter Verwendung von die Biegbarkeit des medizinischen Instruments (2) beschreibenden Eigenschaftsdaten wenigstens eine prädiktive Fortsetzungstrajektorie zu ermitteln, und
- eine Ausgabeeinheit (15) zur Ausgabe einer auf den Bilddaten (32) basierenden Darstellung (31) des Eingriffsbereichs und der wenigstens einen Fortsetzungstrajektorie auf der Darstellungseinrichtung (9); und
- eine Lichtführungseinrichtung (7) zur Projektion wenigstens eines Lichtmusters auf die Projektionsfläche (17) derart, dass durch Zusammenschau wenigstens einer der wenigstens einen Markierung und des Lichtmusters ein Positionierhinweis zur manuellen Positionskorrektur an dem proximalen Anteil (21) des medizinischen Instruments (2) zum Erreichen einer auszuwählenden der wenigstens einen Fortsetzungstrajektorie bei weiterem Einschub des medizinischen Instruments (2) entsteht.

2. Eingriffsvorrichtung nach Anspruch 1, **dadurch gekennzeichnet, dass** das Lichtmuster einen Projektionspunkt (40) markiert, der zur Auswahl der ausgewählten Fortsetzungstrajektorie mit einer der ausgewählten Fortsetzungstrajektorie zugeordneten Markierung in Einklang zu bringen ist, wobei die Ausgabeeinheit (15) zur Erzeugung der Darstellung (31) mit der wenigstens einen Fortsetzungstrajektorie zugeordneten, die zugeordnete Markierung beschreibenden Markierungsinformationen ausgebildet ist.

3. Eingriffsvorrichtung nach Anspruch 1 oder 2, **dadurch gekennzeichnet, dass** das Lichtmuster wenigstens eine die Orientierung der Darstellung (31) anzeigende Orientierungslinie (37) umfasst, insbesondere eine Bildebene einer in der Darstellung (31) verwendeten Schicht der Bilddaten (32).

4. Eingriffsvorrichtung nach Anspruch 2 und 3, **dadurch gekennzeichnet, dass** das Lichtmuster zur Markierung des Projektionspunkts (40) auf der Orientierungslinie (37) einen, insbesondere durch die Verarbeitungseinrichtung (11) aufgrund der Positionierungsinformation ansteuerbar einstellbaren, Orientierungspunkt (45) umfasst.

5. Eingriffsvorrichtung nach Anspruch 3 oder 4, **dadurch gekennzeichnet, dass** die Markierungen wenigstens eine mit der Orientierungslinie (37) in Deckung zu bringende Ausrichtungslinie (26) aufweisen.

6. Eingriffsvorrichtung nach Anspruch 5, **dadurch gekennzeichnet, dass** Fortsetzungstrajektorien zuordenbare Markierungen entlang der Ausrichtungslinie (26) vorgesehen sind, so dass bei mit der Orientierungslinie (37) in Deckung gebrachter Ausrichtungslinie (26) für jede Positionskorrektur das medizinische Instrument (2) in einer durch die Orientierungslinie (37) markierten Schicht verbleibt.

7. Eingriffsvorrichtung nach Anspruch 5 oder 6, **dadurch gekennzeichnet, dass**
- die Markierungen wenigstens einen Teil eines die wenigstens eine Ausrichtungslinie (26) umfassenden Fadenkreuzes bilden, dessen Mitte vor Vornahme der Positionskorrektur mit dem Projektionspunkt (40) übereinstimmt, und dessen Ringe (27, 28, 29), insbesondere in unterschiedlichen Farben, jeweiligen Fortsetzungstrajektorien zugeordnet sind, und/oder
- die Markierungen ein, insbesondere matrixartiges, Koordinatenraster bilden, wobei zumindest den Fortsetzungstrajektorien zuordenbaren Markierungen Koordinaten zugeordnet sind.

8. Eingriffsvorrichtung nach Anspruch 1, **dadurch gekennzeichnet, dass** das Ausrichtungselement (3) nur eine einen Ausrichtpunkt (48) definierende Markierung aufweist und die Verarbeitungseinrichtung (11) ausgebildet ist, die Lichtführungseinrichtung (7) zur Markierung eines einer Fortsetzungstrajektorie zugeordneten Projektionspunkts (49) auf der Projektionsfläche (17) anzusteuern, der zur Auswahl der zugeordneten Fortsetzungstrajektorie mit dem Ausrichtpunkt (48) in Einklang zu bringen ist.

9. Eingriffsvorrichtung nach einem der vorangehenden Ansprüche, **dadurch gekennzeichnet, dass** der proximale Anteil (21) einen Angriffspunkt zur manuellen Vornahme der Positionskorrektur aufweist, wobei die Auswertungseinheit (14) zur Berücksichtigung der Lage des Angriffspunktes bei der Ermittlung der wenigstens einen Fortsetzungstrajektorie ausgebildet ist.

10. Eingriffsvorrichtung nach einem der vorangehenden Ansprüche, **dadurch gekennzeichnet, dass** sie eine Bildgebungseinrichtung (5) zur Aufnahme wenigstens eines Teils der Bilddaten (32) aufweist.

11. Eingriffsvorrichtung nach Anspruch 10, **dadurch gekennzeichnet, dass** die Lichtführungseinrichtung (7) an der Bildgebungseinrichtung (5) fest verbaut und/oder in diese integriert ist.

12. Eingriffsvorrichtung nach einem der vorangehenden Ansprüche, **dadurch gekennzeichnet, dass** die Auswertungseinheit (14) zur Ermittlung der Positionierungsdaten wenigstens teilweise aus den Bilddaten (32) ausgebildet ist und/oder zur Registrierung der Bilddaten (32) mit bereitgestellten Vorab-Bilddaten des Untersuchungsobjekts (19) ausgebildet ist.

13. Eingriffsvorrichtung nach einem der vorangehenden Ansprüche, **dadurch gekennzeichnet, dass** die Auswertungseinheit (14) zur Ermittlung der Eigenschaftsdaten aus einer bereitgestellten Instrumenteninformation ausgebildet ist.

14. Computerimplementiertes Verfahren zur Ermittlung und Ausgabe eines Positionierhinweises für ein medizinisches, längliches Instrument (2), das für einen Eingriff in einen Eingriffsbereich eines Untersuchungsobjekts (19) teilweise einführbar ist, einen proximalen, außerhalb des Untersuchungsobjekts (19) anzuordnenden Anteil (21) zur manuellen Handhabung des medizinischen Instruments (2) aufweist und wenigstens teilweise biegbar ist, wobei ein an dem Instrument (2) angeordnetes und/oder in dieses integriertes Ausrichtungselement (3), das eine Projektionsfläche (17) mit wenigstens einer Markierung, die wenigstens einen Punkt markiert, verwendet wird, wobei das Verfahren bei bereits teilweise in das Untersuchungsobjekt (19) eingeführtem Instrument (2) folgende Schritte aufweist:
- Bereitstellen von Bilddaten (32) des Untersuchungsobjekts (19), die den Eingriffsbereich darstellen, über eine Schnittstelle einer Verarbeitungseinrichtung (11) und Ermittlung von Positionierungsdaten, die die aktuelle Position und Lage des medizinischen Instruments (2) beschreiben,
- mittels einer Auswertungseinheit (14) der Verarbeitungseinrichtung (11), Ermittlung wenigstens einer prädiktiven Fortsetzungstrajektorie aus den Bilddaten (32) und den Positionierungsdaten unter Verwendung von die Biegbarkeit des medizinischen Instruments (2) beschreibenden Eigenschaftsdaten,
- Ausgabe einer auf den Bilddaten (32) basierenden Darstellung (31) des Eingriffsbereichs und der wenigstens einen Fortsetzungstrajektorie mittels einer Ausgabeeinheit (15) der Verarbeitungseinrichtung (11) an einer Darstellungseinrichtung (9); und
- Betrieb einer Lichtführungseinrichtung (7) zur Projektion wenigstens eines Lichtmusters auf die Projektionsfläche (17) derart, dass durch Zusammenschau wenigstens einer der wenigstens einen Markierung und des Lichtmusters der Positionierhinweis zur manuellen Positionskorrektur an dem proximalen Anteil (21) des medizinischen Instruments (2) zum Erreichen einer auszuwählenden der wenigstens einen Fortsetzungstrajektorie bei weiterem Einschub des medizinischen Instruments (2) entsteht.

15. Computerprogramm, welches, wenn es auf einer Verarbeitungseinrichtung (11) einer Eingriffsvorrichtung (1) ausgeführt wird, diese veranlasst, die Schritte eines Verfahrens nach Anspruch 14 auszuführen.

## Claims

1. Medical intervention device (1), having
- an elongate medical instrument (2), which can be partially inserted into an intervention area of an examination object (19) for an intervention, has a proximal portion (21) to be arranged outside the examination object (19) for manual handling of the medical instrument (2) and is at least partially bendable,
- an alignment element (3) arranged on the instrument (2) and/or integrated therein, which has a projection surface (17) with at least one marking which marks at least one point,
- a representation facility (9),
**characterised in that** the medical intervention device has:
- a processing facility (11) which has:
- an evaluation unit (14) which is embodied to ascertain at least one predictive continuation trajectory from image data (32) of the examination object (19) representing the intervention area and positioning data describing the current position and location of the medical instrument (2) using property data describing the bendability of the medical instrument (2), and
- an output unit (15) for outputting a representation (31) of the intervention area and the at least one continuation trajectory based on the image data (32) on the representation facility (9); and
- a light-guiding facility (7) for projecting at least one light pattern onto the projection surface (17) in such a way that a combined view of at least one of the at least one marking and the light pattern produces a positioning instruction for manual position correction at the proximal portion (21) of the medical instrument (2) in order to achieve one to be selected of the at least one continuation trajectory upon further insertion of the medical instrument (2).

2. Intervention device according to claim 1, **characterised in that** the light pattern marks a projection point (40) which, in order to select the selected continuation trajectory, is to be brought into congruence with a marking assigned to the selected continuation trajectory, wherein the output unit (15) is embodied to generate the representation (31) with marking information assigned to the at least one continuation trajectory and describing the assigned marking.

3. Intervention device according to claim 1 or 2, **characterised in that** the light pattern comprises at least one orientation line (37) indicating the orientation of the representation (31), in particular an image plane of a slice of the image data (32) used in the representation (31).

4. Intervention device according to claim 2 and 3, **characterised in that** the light pattern for marking the projection point (40) on the orientation line (37) comprises an orientation point (45), which can in particular be controllably adjusted by the processing facility (11) on the basis of the positioning information.

5. Intervention device according to claim 3 or 4, **characterised in that** the markings have at least one alignment line (26) to be brought into coincidence with the orientation line (37).

6. Intervention device according to claim 5, **characterised in that** markings that can be assigned to continuation trajectories are provided along the alignment line (26), so that, when the alignment line (26) is brought into coincidence with the orientation line (37), for each position correction, the medical instrument (2) remains in a slice marked by the orientation line (37).

7. Intervention device according to claim 5 or 6, **characterised in that**
- the markings form at least part of a crosshair comprising the at least one alignment line (26), the centre of which coincides with the projection point (40) before the position correction is undertaken, and the rings (27, 28, 29) of which, in particular in different colours, are assigned to respective continuation trajectories, and/or
- the markings form a coordinate grid, in particular a matrix-like grid, wherein coordinates are assigned at least to markings that can be assigned to the continuation trajectories.

8. Intervention device according to claim 1, **characterised in that** the alignment element (3) has only one marking defining an alignment point (48) and the processing facility (11) is embodied to actuate the light-guiding facility (7) for marking a projection point (49) on the projection surface (17) which is assigned to a continuation trajectory and which, in order to select the assigned continuation trajectory, is to be brought into congruence with the alignment point (48).

9. Intervention device according to one of the preceding claims, **characterised in that** the proximal portion (21) has a point of application for manually undertaking the position correction, wherein the evaluation unit (14) is embodied to take account of the location of the point of application when ascertaining the at least one continuation trajectory.

10. Intervention device according to one of the preceding claims, **characterised in that** it has an imaging facility (5) for capturing at least part of the image data (32).

11. Intervention device according to claim 10, **characterised in that** the light-guiding facility (7) is permanently installed on the imaging facility (5) and/or integrated therein.

12. Intervention device according to one of the preceding claims, **characterised in that** the evaluation unit (14) is embodied to ascertain the positioning data at least partially from the image data (32) and/or to register image data (32) with provided preliminary image data of the examination object (19).

13. Intervention device according to one of the preceding claims, **characterised in that** the evaluation unit (14) is embodied to ascertain the property data from provided instrument information.

14. Computer-implemented method for ascertaining and outputting a positioning instruction for an elongate medical instrument (2), which can be partially inserted into an intervention area of an examination object (19) for an intervention, has a proximal portion (21) to be arranged outside the examination object (19) for manual handling of the medical instrument (2) and is at least partially bendable, wherein an alignment element (3) arranged on the instrument (2) and/or integrated therein, which has a projection surface (17) with at least one marking which marks at least one point is used, wherein, with an instrument (2) that is already partially inserted into the examination object (19), the method has the following steps:
- providing image data (32) of the examination object (19) representing the intervention area via an interface of a processing facility (11) and ascertaining positioning data describing the current position and location of the medical instrument (2),
- using an evaluation unit (14) of the processing facility (11) to ascertain at least one predictive continuation trajectory from the image data (32) and the positioning data using property data describing the bendability of the medical instrument (2),
- outputting a representation (31) of the intervention area and the at least one continuation trajectory based on the image data (32) by means of an output unit (15) of the processing facility (11) on a representation facility (9); and
- operating a light-guiding facility (7) for projecting at least one light pattern onto the projection surface (17) in such a way that a combined view of at least one of the at least one marking and the light pattern produces the positioning instruction for manual position correction at the proximal portion (21) of the medical instrument (2) in order to achieve one to be selected of the at least one continuation trajectory upon further insertion of the medical instrument (2).

15. Computer program, which, when executed on a processing facility (11) of an intervention device (1), causes it to execute the steps of a method according to claim 14.

## Revendications

1. Installation (1) médicale d'intervention, comportant
- un instrument (2) médical oblong, qui peut être introduit au moins en partie pour une intervention dans une partie d'intervention d'un objet (19) à examiner, qui a, pour la manipulation manuelle de l'instrument (2) médical, une partie (21) proximale pouvant être mise à l'extérieur de l'objet (19) à examiner, et qui peut se courber au moins en partie,
- un élément (3) d'orientation monté sur l'instrument (2) et/ou intégré dans celui-ci, qui a une surface (17) de projection ayant au moins un repère, qui repère au moins un point,
- un dispositif (9) de représentation,
**caractérisée en ce que** l'installation médicale d'intervention a :
- un dispositif (11) de traitement, qui a
- une unité (14) d'évaluation, qui est constituée pour déterminer, à partir de données (32) d'image représentant la partie d'intervention de l'objet (19) à examiner et de données de positionnement décrivant l'endroit en cours et la position de l'instrument (2) médical en utilisant des données de propriété décrivant la flexibilité de l'instrument (2) médical, au moins une trajectoire de continuation prévisible, et
- une unité (15) de sortie pour la sortie d'une représentation (31), reposant sur les données (32) d'image, de la partie d'intervention et de la au moins une trajectoire de continuation, sur le dispositif (9) de représentation ; et
- un dispositif (7) de conduite de la lumière pour la projection d'au moins un motif lumineux sur la surface (17) de projection, de manière à créer, en voyant ensemble au moins l'un des au moins un repère et du motif lumineux, une indication de positionnement pour la correction manuelle de la position sur la partie (21) proximale de l'instrument (2) médical, afin d'obtenir une trajectoire à choisir de la au moins une trajectoire de continuation lorsque l'on continue à insérer l'instrument (2) médical.

2. Installation d'intervention suivant la revendication 1, **caractérisée en ce que** le motif lumineux repère un point (40) de projection qui, pour la sélection de la trajectoire de continuation choisie, est à mettre en coïncidence avec un repère associé à la trajectoire de continuation choisie, dans laquelle l'unité (15) de sortie est constituée pour la production de la représentation (31) avec les informations de repérage associées à la au moins une trajectoire de continuation et décrivant le repère associé.

3. Installation d'intervention suivant la revendication 1 ou 2, **caractérisée en ce que** le motif lumineux comprend au moins une ligne (37) d'orientation indiquant l'orientation de la représentation (31), en particulier un plan d'image d'une tranche, utilisée dans la représentation (31), des données (32) d'image.

4. Installation d'intervention suivant la revendication 2 et 3, **caractérisée en ce que** le motif lumineux comprend, pour le repérage du point (40) de projection sur la ligne (37) d'orientation, un point (45) d'orientation réglable de manière commandée sur la base de l'information de positionnement, en particulier par le dispositif (11) de traitement.

5. Installation d'intervention suivant la revendication 3 ou 4, **caractérisée en ce que** les repères ont au moins une ligne (26) de direction à mettre en coïncidence avec la ligne (37) d'orientation.

6. Installation d'intervention suivant la revendication 5, **caractérisée en ce qu'**il est prévu, le long de la ligne (26) de direction, des repères pouvant être associés à des trajectoires de continuation, de manière à ce que, lorsque la ligne (26) de direction est mise en coïncidence avec la ligne (37) d'orientation pour chaque correction de position, l'instrument (2) médical reste dans une tranche repérée par la ligne (37) d'orientation.

7. Installation d'intervention suivant la revendication 5 ou 6, **caractérisée en ce que**
- les repères forment au moins une partie d'un réticule comprenant la au moins une ligne (26) de direction dont le milieu coïncide, avant l'exécution de la correction de position, avec le point (40) de projection, et dont les anneaux (27, 28, 29), en particulier de couleurs différentes, sont associés à des trajectoires de continuation respectives, et/ou
- les repères forment une trame de coordonnées, en particulier de type matricielle, dans laquelle au moins des coordonnées sont associées aux repères pouvant être associés aux trajectoires de continuation.

8. Installation d'intervention suivant la revendication 1, **caractérisée en ce que** l'élément (3) d'orientation n'a qu'un repère défini sans un point (48) d'orientation et le dispositif (11) de traitement est constitué pour commander le dispositif (7) de conduite de la lumière pour le repérage sur la surface (17) de projection d'un point (49) de projection associé à une trajectoire de continuation, lequel est, pour la trajectoire de continuation associée, à mettre en coïncidence avec le point (48) d'orientation.

9. Installation d'intervention suivant l'une des revendications précédentes, **caractérisée en ce que** la partie (21) proximale a un point d'attaque pour l'exécution manuelle de la correction de position, dans laquelle l'unité (14) d'évaluation est constituée pour la prise en compte de la position du point d'attaque lors de la détermination de la au moins une trajectoire de continuation.

10. Installation d'intervention suivant l'une des revendications précédentes, **caractérisée en ce qu'**elle a un dispositif (5) d'imagerie pour l'enregistrement d'au moins une partie des données (32) d'image.

11. Installation d'intervention suivant la revendication 10, **caractérisée en ce que** le dispositif (7) de conduite de la lumière est fixé sur le dispositif (5) d'imagerie et/ou y est intégré.

12. Installation d'intervention suivant l'une des revendications précédentes, **caractérisée en ce que** l'unité (14) d'évaluation est constituée pour la détermination des données de positionnement au moins en partie à partir des données (32) d'image et/ou pour la mise en correspondance des données (32) d'image avec des données d'image précédentes mises à disposition de l'objet (19) à étudier.

13. Installation d'intervention suivant l'une des revendications précédentes, **caractérisée en ce que** l'unité (14) d'évaluation est constituée pour la détermination des propriétés à partir d'une information d'instrument mise à disposition.

14. Procédé mis en œuvre par ordinateur de détermination et de sortie d'une indication de positionnement d'un instrument (2) médical oblong, qui peut être introduit en partie pour une intervention dans une partie d'intervention d'un objet (19) à examiner, qui a une partie (21) proximale disposée à l'extérieur de l'objet (19) à examiner pour la manipulation manuelle de l'instrument (2) médical, et qui peut se courber au moins en partie, dans lequel on utilise un élément (3) d'orientation monté sur l'instrument (2) et/ou y étant intégré, qui a une surface (17) de projection ayant au moins un repère, qui repère au moins un point, dans lequel le procédé a, alors que l'instrument (2) est déjà introduit en partie dans l'objet (19) à examiner, les stades suivants :
- se procurer des données (32) d'image de l'objet (19) à examiner, qui représentent la partie d'intervention par une interface d'un dispositif (11) de traitement et déterminer des données de positionnement, qui décrivent l'emplacement et la position en cours de l'instrument (2) médical,
- au moyen d'une unité (14) d'évaluation du dispositif (11) de traitement, déterminer au moins une trajectoire prévisible de continuation à partir des données (32) d'image et des données de positionnement en utilisant des données de propriété décrivant la flexibilité de l'instrument (2) médical,
- sortie, sur un dispositif (9) de représentation, au moyen d'une unité (15) de sortie du dispositif (11) de traitement, d'une représentation (31), reposant sur les données (32) d'image, de la partie d'intervention et d'au moins une trajectoire de continuation ; et
- mettre en fonctionnement d'un dispositif (7) de conduite de la lumière pour la projection d'au moins un motif lumineux sur la surface (17) de projection, de manière à créer, en voyant ensemble au moins l'un des au moins un repère et du motif lumineux, une indication de positionnement pour la correction manuelle de la position sur la partie (21) proximale de l'instrument (2) médical, afin d'obtenir une trajectoire à choisir de la au moins une trajectoire de continuation, lorsque l'on continue à insérer l'instrument (2) médical.

15. Programme d'ordinateur qui, lorsqu'il est exécuté sur un dispositif (11) de traitement d'une installation (1) d'intervention, fait que celle-ci exécute les stades d'un procédé suivant la revendication 14.
